# EUROPEAN PATENT APPLICATION

(11) **EP 4 553 073 A1**
(43) Date of publication of application: **14.05.2025**
(21) Application number: 23834940.1
(22) Date of filing: 07.07.2023
(51) Int. Cl.: C07D 405/14, A61K 31/4427, A61P 3/00, A61P 25/00

(54) **SALT OF GLP-1R AGONIST, PREPARATION METHOD THEREFOR AND USE THEREOF**

(30) Priority: 07.07.2022 CN 202210804212
(71) Applicant: Mindrank AI Ltd., Hangzhou, Zhejiang 310018 (CN)
(72) Inventor: ZHANG, Long, Hangzhou, China (Zhejiang) Pilot Free Trade Zone, Hangzhou, Zhejiang 310018 (CN); NIU, Zhangming, Hangzhou, China (Zhejiang) Pilot Free Trade Zone, Hangzhou, Zhejiang 310018 (CN); HU, Yang, Hangzhou, China (Zhejiang) Pilot Free Trade Zone, Hangzhou, Zhejiang 310018 (CN)
(74) Representative: Gille Hrabal Partnerschaftsgesellschaft mbB Patentanwälte
(86) International application number: PCT/CN2023/106237
(87) International publication number: WO 2024/008174

(57) **Abstract**

The present invention relates to a salt form of compound I, which is a GLP-1R agonist, a preparation method therefor, and use thereof. The salt of the present invention has excellent GLP-1R agonistic activity and has good bioavailability in animals, thus being suitable for preparing a preparation for treating metabolic diseases, tumors, autoimmune diseases, or metastatic diseases.

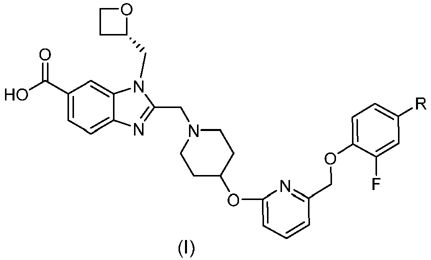

## Description

The present disclosure claims priority to a prior application filed with China National Intellectual Property Administration on July 7, 2022, having the patent application number 202210804212X, and entitled "SALT OF GLP-1R AGONIST, PREPARATION METHOD THEREFOR AND USE THEREOF", which is incorporated herein by reference in its entirety.

### TECHNICAL FIELD

The present disclosure pertains to the field of drug development and particularly relates to a salt of a GLP-1R agonist, a preparation method therefor, and use thereof.

### BACKGROUND

Diabetes is a chronic disease characterized by high blood glucose levels and is caused by (relatively or absolutely) insufficient insulin secretion or insulin action disorders. According to the ninth edition (the latest edition) of the International Diabetes Federation (IDF) Diabetes Atlas, approximately 463 million adults (aged 20-79) worldwide were living with diabetes in 2019, and the number of patients with diabetes is projected to be 578 million by 2030. At this rate, 700 million people worldwide will be living with diabetes in 2045. Therefore, diabetes has become one of the most pressing global social health problems of the 21st century.

Currently, various pharmacological methods are available to treat hyperglycemia and the attendant T2DM (Hampp et al., Use of Antidiabetic Drugs in the U.S., 2003-2012, Diabetes Care, 37:1367-1374, 2014). These methods can be classified into six major categories, each acting through a different major mechanism.

Insulin secretagogues, including sulfonylureas, dipeptidyl peptidase IV (PP-IV) inhibitors, and glucagon-like peptide-1 receptor (GLP-1R) agonists, enhance insulin secretion by acting on pancreatic β cells. Sulfonylureas have limited efficacy and tolerability, cause weight gain, and often induce hypoglycemia. DP-IV inhibitors have limited efficacy. Commercially available GLP-1R agonists are peptides that are administered by subcutaneous injection. Liraglutide is additionally approved for the treatment of obesity.

Biguanides, such as metformin, are believed to act mainly by reducing hepatic glucose production. Biguanides often cause gastrointestinal discomfort and lactic acidosis, which further limit their use.

α-Glucosidase inhibitors, such as acarbose, can reduce intestinal glucose absorption. These medicaments often cause gastrointestinal discomfort.

Thiazolidinediones, such as pioglitazone and rosiglitazone, act on specific receptors in the liver, muscle, and adipose tissue. They regulate lipid metabolism and subsequently enhance the responses of these tissues to insulin action. Frequent use of these drugs may cause weight gain and may induce edema and anemia.

Insulin, either alone or in combination with the medicaments described above, is used for more severe cases, and frequent use of insulin may also cause weight gain and involves a risk of hypoglycemia.

Sodium-glucose cotransporter 2 (SGLT2) inhibitors, such as dapagliflozin, empagliflozin, canagliflozin, and ertugliflozin, inhibit glucose reabsorption in the kidneys, thereby lowering blood glucose levels. This emerging drug may be associated with ketoacidosis and urinary tract infections.

However, except for GLP-1R agonists and SGLT2 inhibitors, these drugs have limited efficacy and fail to address the most important issues: the functional decline of β cells and related obesity. Therefore, there is a need for more effective pharmaceuticals with relatively few side effects and easy administration.

GLP-1 is a 30-amino-acid-long incretin hormone secreted by intestinal L cells in response to food intake. GLP-1 has been shown to stimulate insulin secretion in a physiological and glucose-dependent manner, reduce glucagon secretion, inhibit gastric emptying, reduce appetite, and stimulate β cell proliferation. In non-clinical trials, GLP-1 promotes continued β cell competence by stimulating transcription of genes important for glucose-dependent insulin secretion and by promoting β cell neogenesis (Meier, et al., Biodrugs, 17(2):93-102, 2013).

In healthy individuals, GLP-1 plays an important role in regulating postprandial blood glucose levels by stimulating glucose-dependent insulin secretion by the pancreas to increase peripheral glucose absorption. GLP-1 also inhibits glucagon secretion, resulting in reduced hepatic glucose output. In addition, GLP-1 delays gastric emptying and slows small bowel movements to delay food absorption. In people with T2DM, the normal postprandial rise in GLP-1 is absent or reduced (Vilsbol1 et al., diabetes, 50: 609-613, 2001).

The structure of GLP-1 has been correspondingly engineered and modified in scientific research to increase its half-life and thus prolong its *in vivo* biological effect. However, currently, clinically used long-acting GLP-1 analogs, such as liraglutide, exenatide, etc., are all polypeptides and require frequent injections, which lead to relatively poor patient compliance. Therefore, the development of small-molecule GLP-1R agonists will be aimed at improving patient compliance, simplifying administration, and reducing side effects of drugs; these agonists have a wide clinical market prospect.

Hangzhou Mindrank AI Ltd. has developed a class of structurally novel small-molecule compounds with inhibitory effects on GLP-1R. The structures of the representative compounds I-1 and I-2 are shown below:

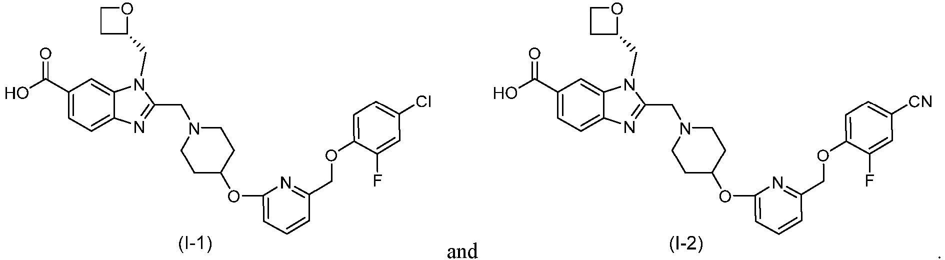

These compounds can significantly enhance the agonism of the GLP-1R target, improve the therapeutic window, and reduce clinical toxic and side effects, meeting the current needs for diabetes treatment worldwide.

The chemical name of compound I-1 is (*S*)-2-(4-(6-(4-chloro-2-fluorophenoxy)methyl)pyridin-2-yl)oxy)piperidin-1-yl)methyl)-1-(oxetan-2-ylmethyl)-1*H-*benzo[*d*]imidazole-6-carboxylic acid.

The chemical name of compound I-2 is (*S*)-2-(4-(6-(4-cyano-2-fluorophenoxy)methyl)pyridin-2-yl)oxy)piperidin-1-yl)methyl)-1-(oxetan-2-ylmethyl)-1*H*-b enzo[*d*]imidazole-6-carboxylic acid.

The successful development of pharmaceutical solid forms of compound I-1 or compound I-2 often requires such properties as having a solid form that is easy to separate and purify after synthesis and a dosage form that is suitable for mass production; can be stored for a relatively long time; absorbs water, decomposes, or converts to other solid forms to a minimal extent; and is suitable for being rapidly absorbed after being administered to an individual (for example, soluble in water and gastric juice).

To meet the needs of clinical research and launching pharmaceutical formulations, it is imperative to develop a solid drug form that is easy to separate and purify, is suitable for industrial production, and has stable physicochemical properties.

### SUMMARY

To solve the problems with the prior art, the present disclosure provides, in a first aspect, a pharmaceutically acceptable salt of a compound represented by formula (I), 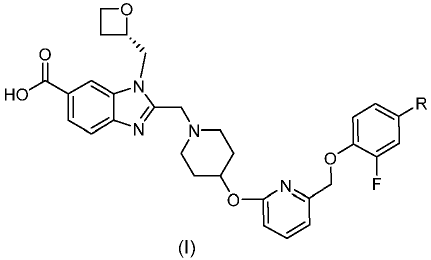 wherein R is selected from halogen and CN.

According to an embodiment of the present disclosure, the compound represented by formula (I) is selected from the following compound I-1 and compound I-2.

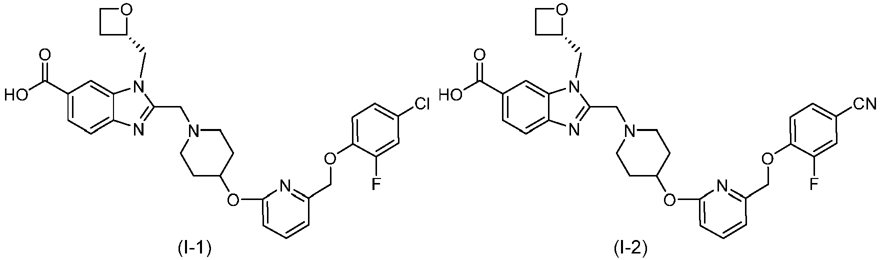

According to an embodiment of the present disclosure, the pharmaceutically acceptable salt refers to a pharmaceutically non-toxic acid addition salt or base addition salt.

According to an embodiment of the present disclosure, the acid addition salt is a salt formed by the compound represented by formula (I) with an inorganic or organic acid, including a hydrobromide, a hydrochloride, a sulfate, a bisulfate, a sulfite, a phosphate, a borate, an acetate, an oxalate, a valerate, a benzoate, a lactate, a toluate, a citrate, a malate, a maleate, a fumarate, a succinate, a tartrate, a methanesulfonate, a benzenesulfonate, and a p-toluenesulfonate; more preferably, the acid addition salt is a hydrochloride, an acetate, a citrate, a malate, a succinate, a tartrate, a fumarate, a maleate, or a methanesulfonate; particularly, the acid addition salt is a citrate or a maleate.

According to an embodiment of the present disclosure, the base addition salt is a salt formed by the compound represented by formula (I) with an inorganic or organic base, including salts formed with alkali metals, such as a sodium salt, a lithium salt, a potassium salt, a calcium salt, a magnesium salt, and the like, and amine salts, including salts formed with ammonia (NH₃), primary amines, secondary amines, or tertiary amines, such as a tetramethylamine salt, a tetraethylamine salt, a methylamine salt, a dimethylamine salt, a trimethylamine salt, a triethylamine salt, an ethylamine salt, a meglumine salt, a choline salt, and a tromethamine salt; more preferably, the base addition salt is a sodium salt, a potassium salt, a calcium salt, a magnesium salt, a meglumine salt, a choline salt, or a tromethamine salt; particularly, the base addition salt is a sodium salt, a potassium salt, a magnesium salt, a meglumine salt, or a tromethamine salt.

According to an embodiment of the present disclosure, an acid addition salt of compound I-1 is a hydrochloride, a tartrate, a maleate, a methanesulfonate, or a citrate; and an acid addition salt of compound I-2 is a citrate, a tartrate, a malate (e.g., an *L*-malate), a fumarate, a methanesulfonate, or a maleate.

According to an embodiment of the present disclosure, a base addition salt of compound I-1 is a sodium salt, a potassium salt, a meglumine salt, or a tromethamine salt; and a base addition salt of compound I-2 is a sodium salt, a potassium salt, a calcium salt, a magnesium salt, a meglumine salt, or a tromethamine salt.

According to a preferred technical solution of the present disclosure, the present disclosure provides a crystal form A of the citrate of compound I-1, wherein an X-ray powder diffraction (XRPD) pattern of the crystal form comprises peaks at diffraction angles (2θ) of 19.77±0.2°, 16.59±0.2°, 22.47±0.2°, and 20.20±0.2°.

According to a preferred technical solution of the present disclosure, the X-ray powder diffraction (XRPD) pattern of the crystal form A of the citrate of compound I-1 comprises peaks at diffraction angles (20) of 16.59±0.2°, 19.77±0.2°, 22.47±0.2°, 20.20±0.2°, 24.84±0.2°, and 17.51±0.2°.

Preferably, the X-ray powder diffraction pattern of the crystal form A of the citrate has the diffraction angles (20) shown in Table 1, wherein the 2θ angles have a margin of error of ±0.20°:

**Table 1**

| 2θ(°) | Intensity% | 2θ ( ° ) | Intensity% |
|---|---|---|---|
| 6.80 | 34.3 | 18.07 | 52.1 |
| 7.13 | 36.4 | 19.77 | 100.0 |
| 8.75 | 33.6 | 20.20 | 61.4 |
| 11.47 | 48.6 | 21.02 | 22.9 |
| 12.06 | 46.4 | 22.47 | 68.6 |
| 13.81 | 27.9 | 24.84 | 60.7 |
| 16.59 | 82.9 | 27.78 | 40.7 |
| 17.51 | 53.6 | | |

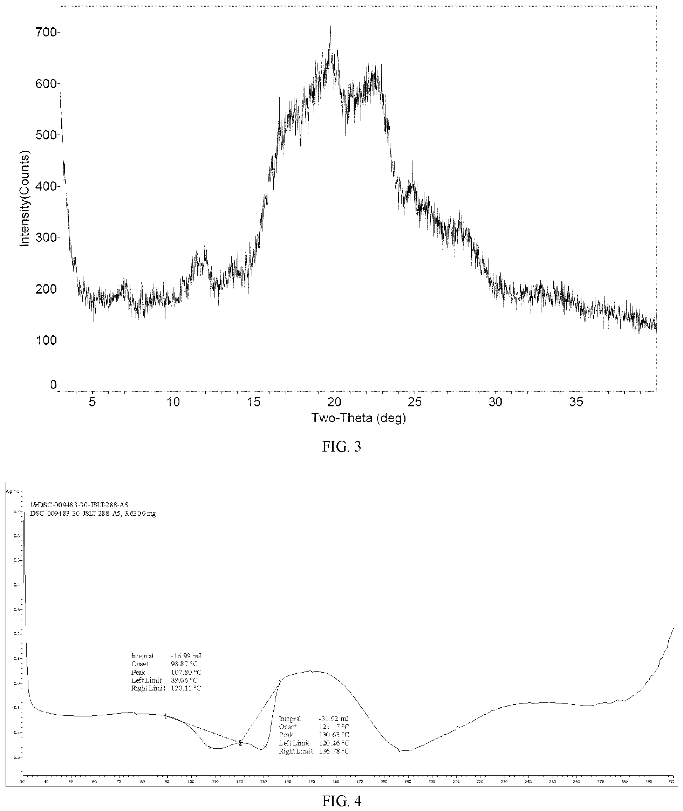

Preferably, the crystal form A of the citrate has the X-ray powder diffraction intensities shown in Table 1. Preferably, the crystal form A of the citrate has an X-ray powder diffraction pattern substantially as shown in FIG. 3.

Preferably, the crystal form A of the citrate has a DSC thermogram with endothermic peaks at temperatures of about 107.80 °C and 130.63 °C.

Preferably, the crystal form A of the citrate has a DSC thermogram substantially as shown in FIG. 4.

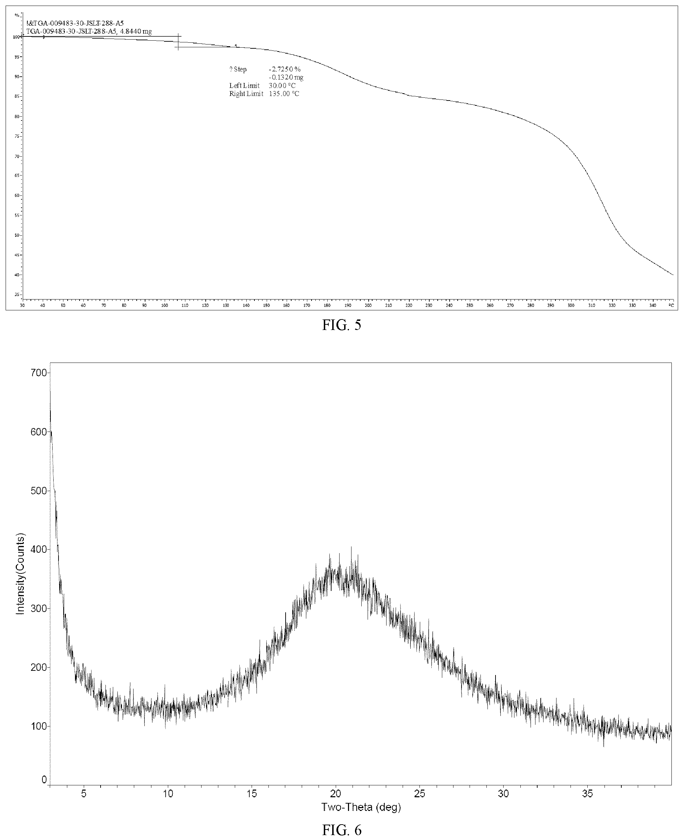

Preferably, the crystal form A of the citrate has a TGA curve substantially as shown in FIG. 5.

As a more preferred embodiment, the present disclosure provides a crystal form A of the sodium salt of compound I-1, wherein an X-ray powder diffraction (XRPD) pattern of the crystal form comprises peaks at diffraction angles (20) of 19.24±0.2°, 20.68±0.2°, 6.81±0.2°, and 14.43±0.2°.

As a more preferred embodiment, the present disclosure provides a crystal form A of the sodium salt of compound I-1, wherein an X-ray powder diffraction (XRPD) pattern of the crystal form comprises peaks at diffraction angles (20) of 19.24±0.2°, 20.68±0.2°, 6.81±0.2°, 14.43±0.2°, 14.984+0.2°, and 6.40±0.2°.

Preferably, the X-ray powder diffraction pattern of the crystal form A of the sodium salt has the diffraction angles (20) shown in Table 2, wherein the 20 angles have a margin of error of ±0.20°:

**Table 2**

| 2θ ( ° ) | Intensity% | 2θ ( ° ) | Intensity% |
|---|---|---|---|
| 4.07 | 36.4 | 18.02 | 40.9 |
| 6.40 | 47.5 | 19.24 | 100.0 |
| 6.81 | 55.0 | 20.68 | 90.1 |
| 14.43 | 53.3 | 24.27 | 34.3 |
| 14.98 | 52.1 | 24.84 | 29.8 |
| 17.69 | 37.6 | 26.44 | 26.4 |

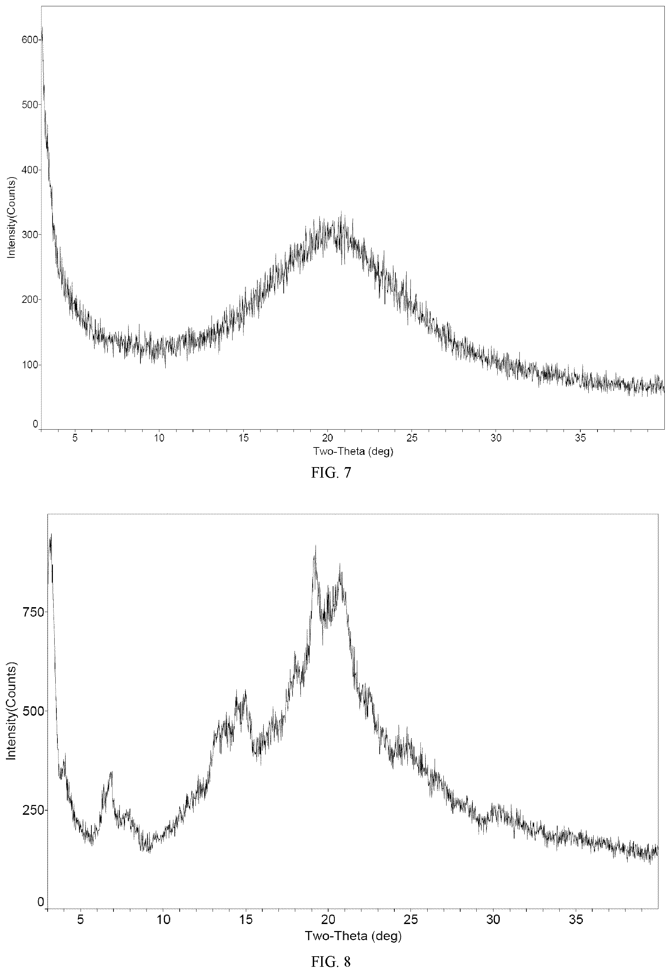

Preferably, the crystal form A of the sodium salt has the X-ray powder diffraction intensities shown in Table 2. Preferably, the crystal form A of the sodium salt has an X-ray powder diffraction pattern substantially as shown in FIG. 8.

Preferably, the crystal form A of the sodium salt has a DSC thermogram with endothermic peaks at temperatures of about 149.11 °C and 174.11 °C.

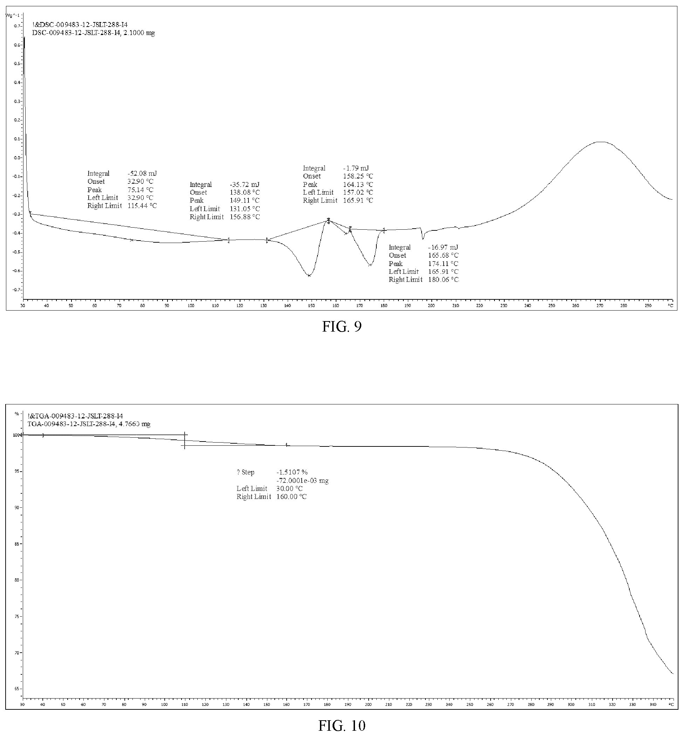

Preferably, the crystal form A of the sodium salt has a DSC thermogram substantially as shown in FIG. 9. Preferably, the crystal form A of the sodium salt has a TGA curve substantially as shown in FIG. 10.

As a more preferred embodiment, the present disclosure provides a crystal form A of the potassium salt of compound I-1, wherein an X-ray powder diffraction (XRPD) pattern of the crystal form comprises peaks at diffraction angles (20) of 13.90±0.2°, 14.43±0.2°, 16.20±0.2°, and 11.67±0.2°.

As a more preferred embodiment, the present disclosure provides a crystal form A of the potassium salt of compound I-1, wherein an X-ray powder diffraction (XRPD) pattern of the crystal form comprises peaks at diffraction angles (20) of 13.90±0.2°, 14.43±0.2°, 16.20±0.2°, 11.67±0.2°, 20.99±0.2°, and 16.79±0.2°.

Preferably, the X-ray powder diffraction pattern of the crystal form A of the potassium salt has the diffraction angles (20) shown in Table 3, wherein the 20 angles have a margin of error of ±0.20°:

**Table 3**

| 2θ ( ° ) | Intensity% | 2θ ( ° ) | Intensity% |
|---|---|---|---|
| 5.80 | 23.5 | 18.95 | 31.1 |
| 6.60 | 36.0 | 20.39 | 33.9 |
| 8.76 | 4.8 | 20.99 | 47.4 |
| 9.99 | 32.7 | 21.95 | 39.3 |
| 11.67 | 49.1 | 23.06 | 29.2 |
| 12.11 | 21.0 | 23.62 | 39.0 |
| 13.42 | 37.7 | 24.91 | 32.8 |
| 13.90 | 100.0 | 25.45 | 15.2 |
| 14.43 | 96.1 | 26.39 | 21.0 |
| 16.20 | 70.6 | 27.80 | 8.3 |
| 16.79 | 45.8 | 32.76 | 7.2 |
| 17.25 | 13.5 | | |

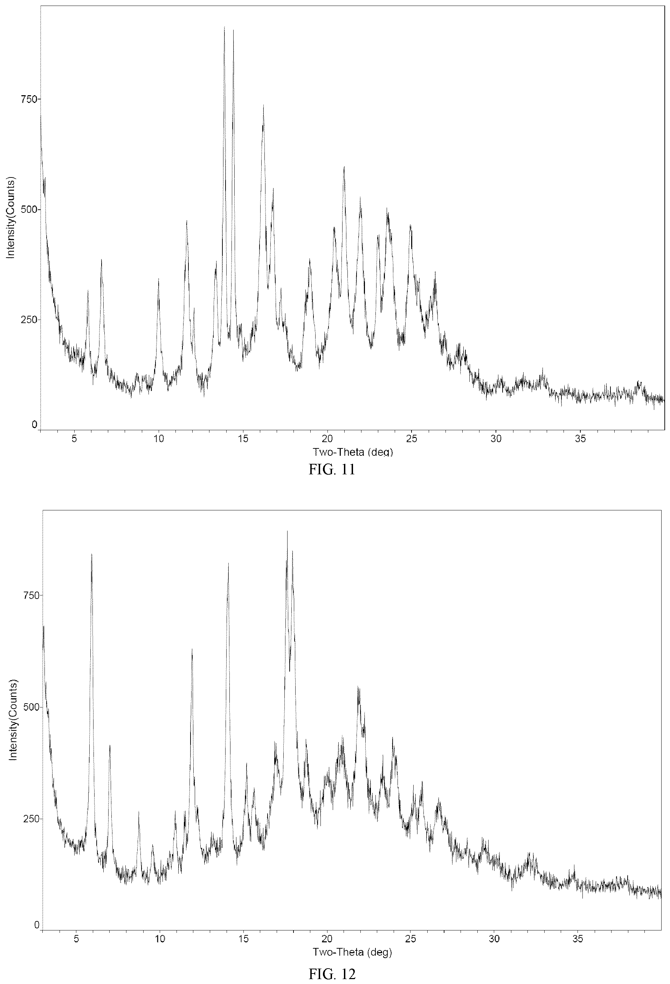

Preferably, the crystal form A of the potassium salt has the X-ray powder diffraction intensities shown in Table 3. Preferably, the crystal form A of the potassium salt has an X-ray powder diffraction pattern substantially as shown in FIG. 11.

As a more preferred embodiment, the present disclosure provides a crystal form B of the potassium salt of compound I-1, wherein an X-ray powder diffraction (XRPD) pattern of the crystal form comprises peaks at diffraction angles (20) of 5.92±0.2°, 14.10±0.2°, 17.62±0.2°, and 17.94±0.2°.

As a more preferred embodiment, the present disclosure provides a crystal form B of the potassium salt of compound I-1, wherein an X-ray powder diffraction (XRPD) pattern of the crystal form comprises peaks at diffraction angles (20) of 5.92±0.2°, 14.10±0.2°, 17.62±0.2°, 17.94±0.2°, 11.92±0.2°, and 7.01±0.2°.

Preferably, the X-ray powder diffraction pattern of the crystal form B of the potassium salt has the diffraction angles (20) shown in Table 4, wherein the 20 angles have a margin of error of ±0.20°:

**Table 4**

| 2θ ( ° ) | Intensity% | 2θ(°) | Intensity% |
|---|---|---|---|
| 5.92 | 100.0 | 20.12 | 11.0 |
| 7.01 | 40.3 | 20.69 | 17.8 |
| 8.74 | 22.1 | 20.90 | 20.8 |
| 9.56 | 11.0 | 21.84 | 39.4 |
| 10.92 | 19.2 | 22.24 | 31.4 |
| 11.92 | 70.3 | 23.33 | 18.4 |
| 12.27 | 17.2 | 24.01 | 23.3 |
| 14.10 | 95.8 | 25.17 | 13.2 |
| 15.19 | 28.1 | 25.69 | 17.4 |
| 15.64 | 19.5 | 26.68 | 15.6 |
| 16.94 | 19.8 | 27.09 | 10.0 |
| 17.62 | 89.6 | 28.38 | 6.7 |
| 17.94 | 80.4 | 29.37 | 8.5 |
| 18.74 | 20.7 | 32.02 | 8.9 |

Preferably, the crystal form B of the potassium salt has the X-ray powder diffraction intensities shown in Table 4. Preferably, the crystal form B of the potassium salt has an X-ray powder diffraction pattern substantially as shown in FIG. 12.

As a more preferred embodiment, the present disclosure provides a crystal form A of the meglumine salt of compound I-1, wherein an X-ray powder diffraction (XRPD) pattern of the crystal form comprises peaks at diffraction angles (20) of 18.15±0.2°, 12.87±0.2°, 22.87±0.2°, and 24.66±0.2°.

As a more preferred embodiment, the present disclosure provides a crystal form A of the meglumine salt of compound I-1, wherein an X-ray powder diffraction (XRPD) pattern of the crystal form comprises peaks at diffraction angles (20) of 18.15±0.2°, 12.87±0.2°, 22.87±0.2°, 24.66±0.2°, 23.21±0.2°, and 19.57±0.2°.

Preferably, the X-ray powder diffraction pattern of the crystal form A of the meglumine salt has the diffraction angles (20) shown in Table 5, wherein the 20 angles have a margin of error of ±0.20°:

**Table 5**

| 2θ ( ° ) | Intensity% | 2θ (°) | Intensity% |
|---|---|---|---|
| 5.221 | 28.9 | 17.26 | 15.5 |
| 7.57 | 9.0 | 18.15 | 100.0 |
| 9.42 | 16.1 | 19.57 | 43.7 |
| 10.23 | 10.0 | 21.39 | 21.9 |
| 10.71 | 20.6 | 22.87 | 55.0 |
| 11.84 | 21.6 | 23.21 | 44.4 |
| 12.87 | 68.1 | 24.66 | 49.5 |
| 15.48 | 16.0 | 25.77 | 13.4 |

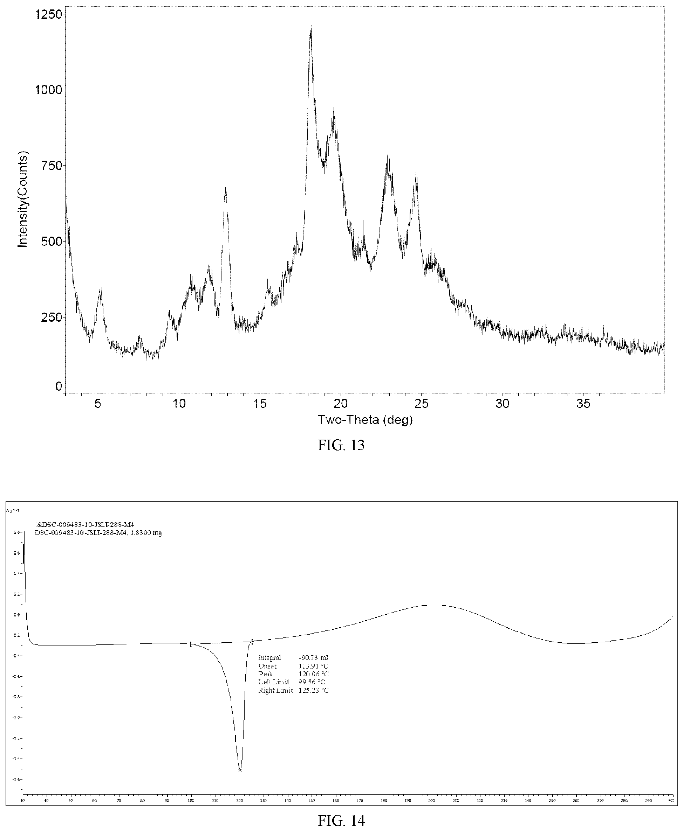

Preferably, the crystal form A of the meglumine salt has the X-ray powder diffraction intensities shown in Table 5. Preferably, the crystal form A of the meglumine salt has an X-ray powder diffraction pattern substantially as shown in FIG. 13.

Preferably, the crystal form A of the meglumine salt has a DSC thermogram with an endothermic peak at a temperature of about 120.06 °C.

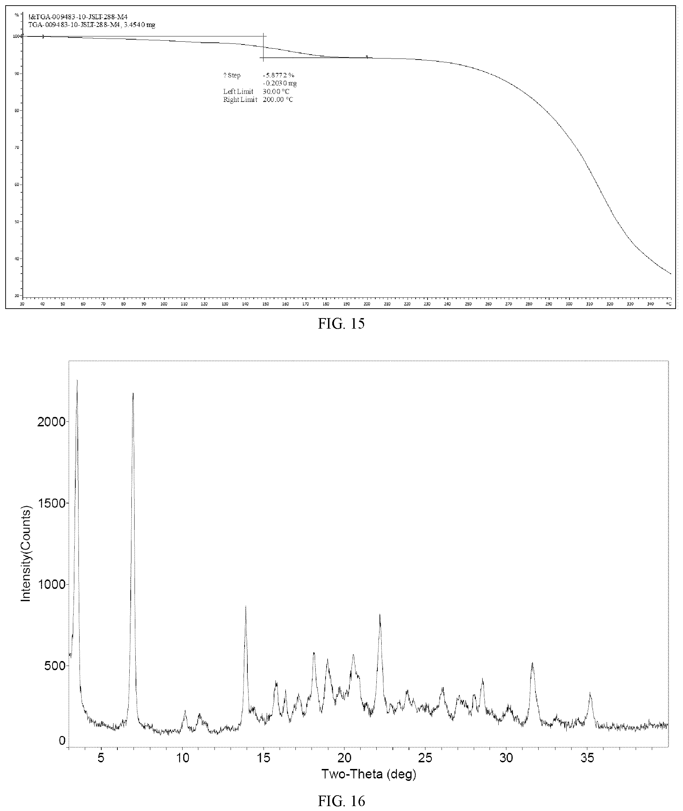

Preferably, the crystal form A of the meglumine salt has a DSC thermogram substantially as shown in FIG. 14. Preferably, the crystal form A of the meglumine salt has a TGA curve substantially as shown in FIG. 15.

As a more preferred embodiment, the present disclosure provides a crystal form A of the tromethamine salt of compound I-1, wherein an X-ray powder diffraction (XRPD) pattern of the crystal form comprises peaks at diffraction angles (20) of 3.50±0.2°, 6.97±0.2°, 13.91±0.2°, and 22.19±0.2°.

As a more preferred embodiment, the present disclosure provides a crystal form A of the tromethamine salt of compound I-1, wherein an X-ray powder diffraction (XRPD) pattern of the crystal form comprises peaks at diffraction angles (20) of 3.50±0.2°, 6.97±0.2°, 13.91±0.2°, 22.19±0.2°, 31.61±0.2°, 18.11±0.2°, and 20.55±0.2°. As the most preferred embodiment, the crystal form A of the tromethamine salt has the X-ray powder diffraction data shown in Table 6 below:

**Table 6**

| 2θ(°) | Intensity% | 2θ ( ° ) | Intensity% |
|---|---|---|---|
| 3.505 | 100.0 | 20.553 | 14.0 |
| 6.972 | 98.6 | 20.864 | 9.0 |
| 10.168 | 6.1 | 22.190 | 28.8 |
| 11.067 | 5.1 | 23.433 | 2.9 |
| 11.490 | 2.8 | 23.885 | 6.0 |
| 13.910 | 35.2 | 24.275 | 3.5 |
| 14.417 | 5.2 | 26.026 | 8.2 |
| 14.924 | 2.3 | 27.098 | 6.1 |
| 15.762 | 11.9 | 27.481 | 4.0 |
| 16.365 | 8.7 | 27.994 | 5.3 |
| 16.911 | 3.9 | 28.520 | 11.0 |
| 17.185 | 6.5 | 30.271 | 5.1 |
| 17.805 | 4.8 | 31.617 | 18.4 |
| 18.118 | 17.3 | 33.113 | 3.4 |
| 18.974 | 13.9 | 35.165 | 9.7 |
| 19.714 | 3.9 | 38.801 | 2.5 |

Preferably, the crystal form A of the tromethamine salt has the X-ray powder diffraction intensities shown in Table 6. Preferably, the crystal form A of the tromethamine salt has an X-ray powder diffraction pattern substantially as shown in FIG. 16.

Preferably, the crystal form A of the tromethamine salt has a DSC thermogram with endothermic peaks at temperatures of about 109.95 °C and 166.02 °C.

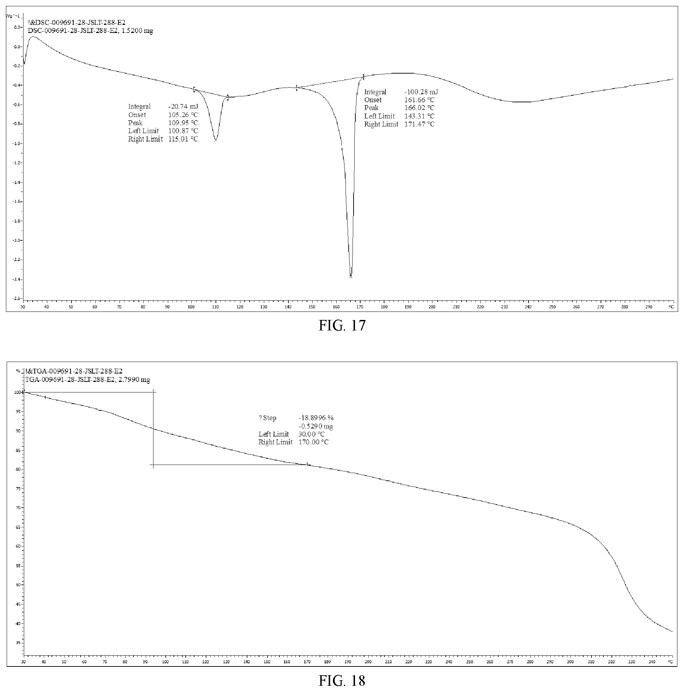

Preferably, the crystal form A of the tromethamine salt has a DSC thermogram substantially as shown in FIG. 17. Preferably, the crystal form A of the tromethamine salt has a TGA curve substantially as shown in FIG. 18. Preferably, the crystal form A of the tromethamine salt is in the form of a N-methylpyrrolidone solvate.

As a more preferred embodiment, the present disclosure provides a crystal form A of the maleate of compound I-2, wherein an X-ray powder diffraction (XRPD) pattern of the crystal form comprises peaks at diffraction angles (2θ) of 5.43±0.2°, 9.89±0.2°, 12.76±0.2°, and 8.30±0.2°.

As a more preferred embodiment, the present disclosure provides a crystal form A of the maleate of compound I-2, wherein an X-ray powder diffraction (XRPD) pattern of the crystal form comprises peaks at diffraction angles (2θ) of 5.43±0.2°, 9.89±0.2°, 12.76±0.2°, 8.30±0.2°, 21.31±0.2°, and 14.24±0.2°.

Preferably, the X-ray powder diffraction pattern of the crystal form A of the maleate has the diffraction angles (20) shown in Table 7, wherein the 2θ angles have a margin of error of ±0.20°:

**Table 7**

| 2θ ( ° ) | Intensity% | 2θ ( ° ) | Intensity% |
|---|---|---|---|
| 5.43 | 100.0 | 18.42 | 5.1 |
| 8.30 | 19.9 | 19.16 | 11.9 |
| 9.15 | 5.6 | 19.62 | 10.5 |
| 9.89 | 23.8 | 20.08 | 4.5 |
| 10.94 | 11.9 | 21.31 | 19.3 |
| 12.76 | 22.0 | 22.09 | 9.8 |
| 14.24 | 16.0 | 24.87 | 10.0 |
| 14.84 | 5.3 | 25.32 | 5.5 |
| 16.22 | 6.2 | 25.77 | 8.1 |
| 16.98 | 10.0 | 28.08 | 5.4 |
| 17.43 | 10.9 | 28.40 | 4.1 |

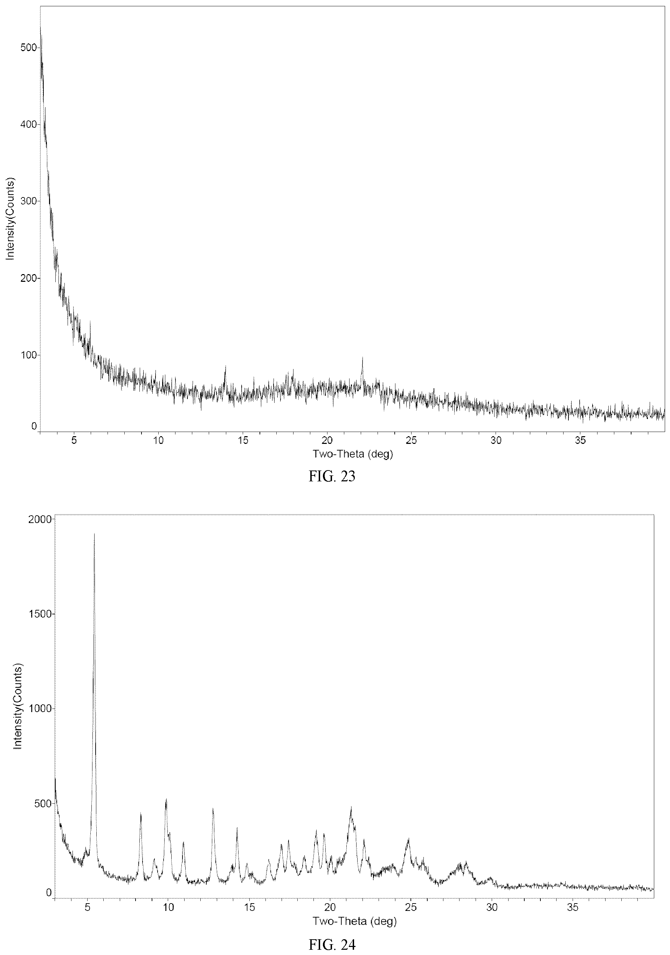

Preferably, the crystal form A of the maleate has the X-ray powder diffraction intensities shown in Table 7. Preferably, the crystal form A of the maleate has an X-ray powder diffraction pattern substantially as shown in FIG. 24.

Preferably, the crystal form A of the maleate has a DSC thermogram with an endothermic peak at a temperature of about 119.30 °C.

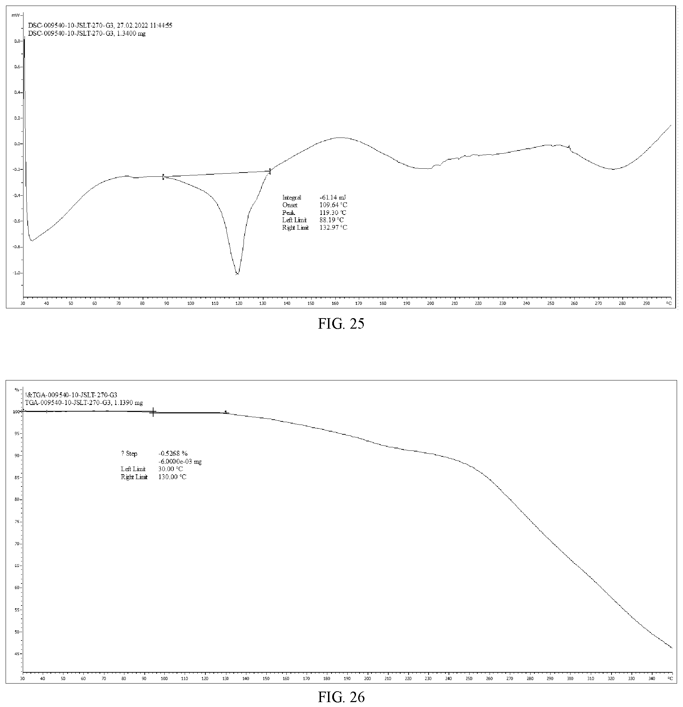

Preferably, the crystal form A of the maleate has a DSC thermogram substantially as shown in FIG. 25.

Preferably, the crystal form A of the maleate has a TGA curve substantially as shown in FIG. 26.

As a more preferred embodiment, the present disclosure provides a crystal form A of the potassium salt of compound I-2, wherein an X-ray powder diffraction (XRPD) pattern of the crystal form comprises peaks at diffraction angles (20) of 11.51±0.2°, 15.42±0.2°, 20.20±0.2°, and 9.52±0.2°.

As a more preferred embodiment, the present disclosure provides a crystal form A of the potassium salt of compound I-2, wherein an X-ray powder diffraction (XRPD) pattern of the crystal form comprises peaks at diffraction angles (20) of 11.51±0.2°, 15.42±0.2°, 20.20±0.2°, 9.52±0.2°, 5.06±0.2°, and 25.38±0.2°.

Preferably, the X-ray powder diffraction pattern of the crystal form A of the potassium salt has the diffraction angles (20) shown in Table 8, wherein the 20 angles have a margin of error of ±0.20°:

**Table 8**

| 2θ ( ° ) | Intensity% | 2θ (°) | Intensity% |
|---|---|---|---|
| 5.06 | 14.2 | 17.41 | 6.1 |
| 7.67 | 11.2 | 19.14 | 3.1 |
| 9.52 | 15.5 | 20.20 | 25.1 |
| 11.51 | 100.0 | 21.45 | 4.8 |
| 13.86 | 4.0 | 25.38 | 12.0 |
| 15.42 | 59.8 | | |

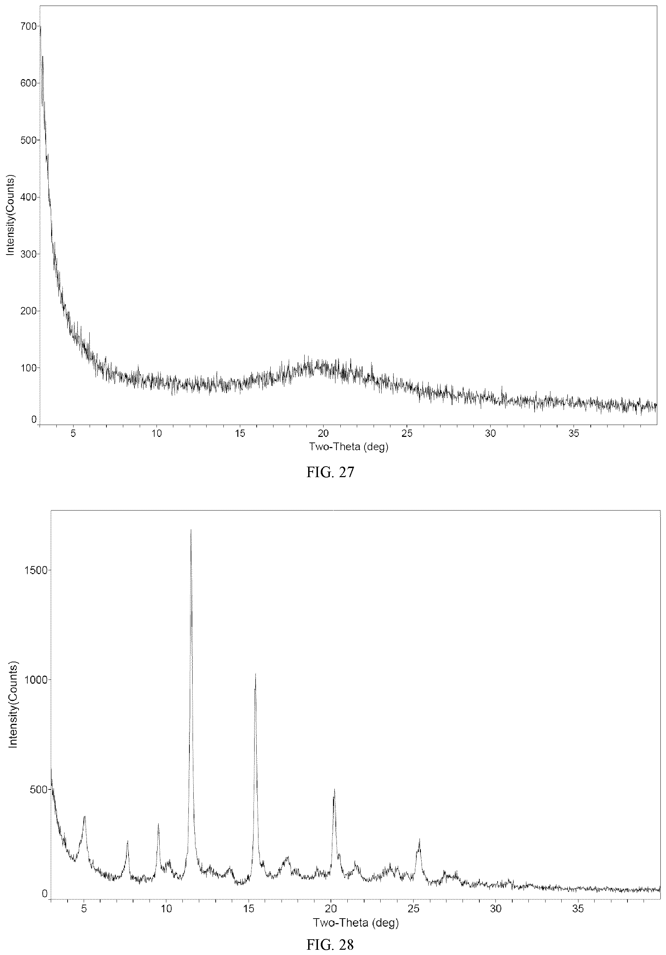

Preferably, the crystal form A of the potassium salt has the X-ray powder diffraction intensities shown in Table 8. Preferably, the crystal form A of the potassium salt has an X-ray powder diffraction pattern substantially as shown in FIG. 28.

Preferably, the crystal form A of the potassium salt has a DSC thermogram with an endothermic peak at a temperature of about 118.44 °C.

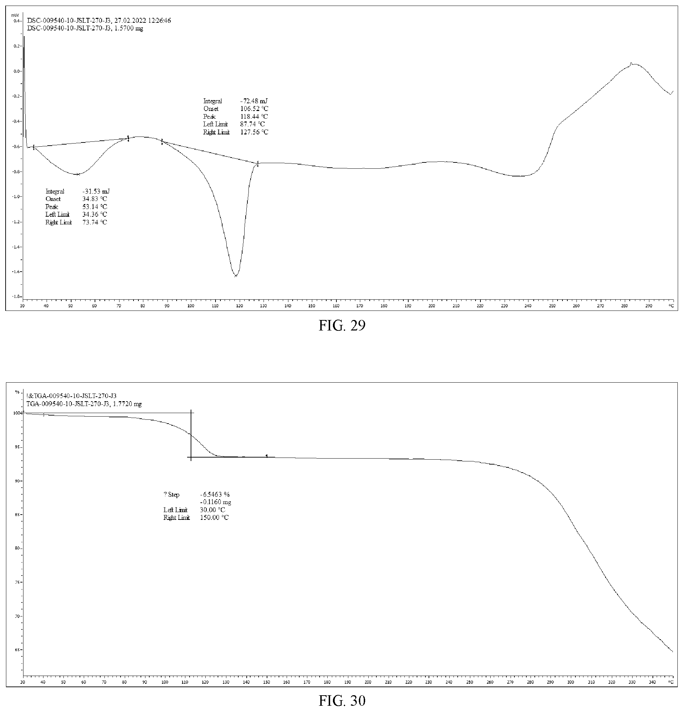

Preferably, the crystal form A of the potassium salt has a DSC thermogram substantially as shown in FIG. 29. Preferably, the crystal form A of the potassium salt has a TGA curve substantially as shown in FIG. 30.

As a more preferred embodiment, the present disclosure provides a crystal form A of the magnesium salt of compound I-2, wherein an X-ray powder diffraction (XRPD) pattern of the crystal form comprises peaks at diffraction angles (20) of 13.92±0.2°, 13.46±0.2°, 14.74±0.2°, and 20.43±0.2°.

As a more preferred embodiment, the present disclosure provides a crystal form A of the magnesium salt of compound I-2, wherein an X-ray powder diffraction (XRPD) pattern of the crystal form comprises peaks at diffraction angles (20) of 13.92±0.2°, 13.46±0.2°, 14.74±0.2°, 20.43±0.2°, 20.16±0.2°, and 17.21±0.2°.

Preferably, the X-ray powder diffraction pattern of the crystal form A of the magnesium salt has the diffraction angles (20) shown in Table 9, wherein the 20 angles have a margin of error of ±0.20°:

**Table 9**

| 2θ ( ° ) | Intensity% | 2θ (°) | Intensity% |
|---|---|---|---|
| 4.55 | 9.6 | 15.84 | 17.8 |
| 6.65 | 4.5 | 16.10 | 10.5 |
| 7.14 | 4.7 | 17.21 | 23.0 |
| 7.87 | 4.2 | 18.76 | 8.7 |
| 9.83 | 5.5 | 20.16 | 23.7 |
| 11.04 | 5.7 | 20.43 | 26.3 |
| 11.63 | 6.0 | 20.92 | 11.3 |
| 12.97 | 7.6 | 22.29 | 6.3 |
| 13.46 | 54.8 | 22.63 | 5.9 |
| 13.92 | 100.0 | 24.21 | 4.5 |
| 14.74 | 28.7 | 25.26 | 11.1 |
| 15.37 | 5.9 | 25.89 | 6.6 |

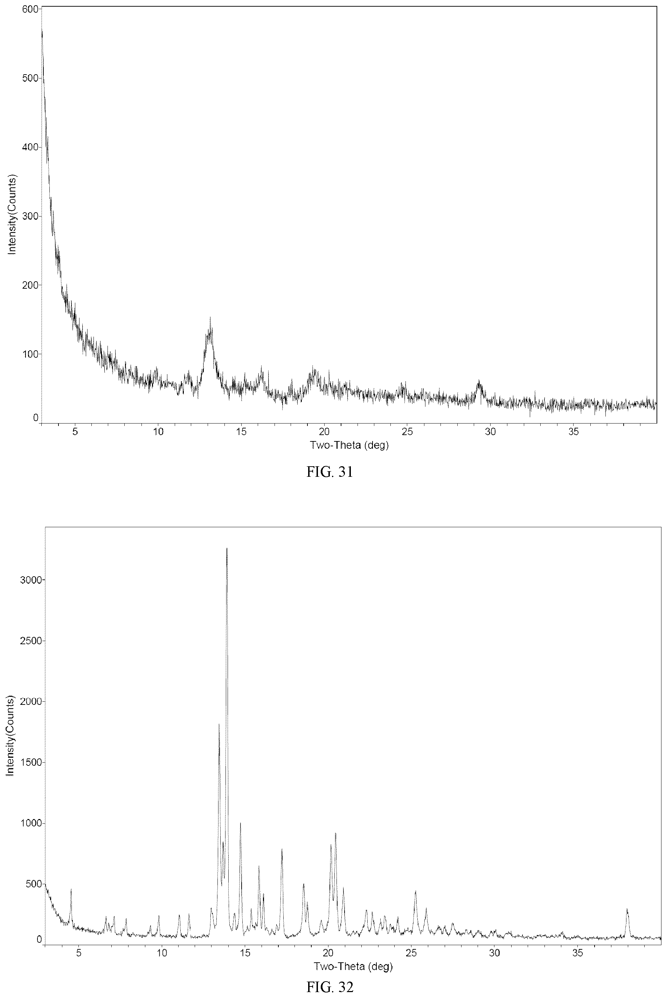

Preferably, the crystal form A of the magnesium salt has the X-ray powder diffraction intensities shown in Table 9. Preferably, the crystal form A of the magnesium salt has an X-ray powder diffraction pattern substantially as shown in FIG. 32.

As a more preferred embodiment, the present disclosure provides a crystal form A of the meglumine salt of compound I-2, wherein an X-ray powder diffraction (XRPD) pattern of the crystal form comprises peaks at diffraction angles (20) of 3.05±0.2°, 9.38±0.2°, 17.62±0.2°, and 12.01±0.2°.

As a more preferred embodiment, the present disclosure provides a crystal form A of the meglumine salt of compound I-2, wherein an X-ray powder diffraction (XRPD) pattern of the crystal form comprises peaks at diffraction angles (20) of 3.05±0.2°, 9.38±0.2°, 17.62±0.2°, 12.01±0.2°, 20.39±0.2°, and 14.88±0.2°.

Preferably, the X-ray powder diffraction pattern of the crystal form A of the meglumine salt has the diffraction angles (20) shown in Table 10, wherein the 2θ angles have a margin of error of +0.20°:

**Table 10**

| 2θ ( ° ) | Intensity% | 2θ (°) | Intensity% |
|---|---|---|---|
| 3.05 | 100.0 | 20.39 | 21.0 |
| 9.38 | 79.6 | 21.71 | 3.7 |
| 12.01 | 21.6 | 22.67 | 4.1 |
| 12.50 | 8.6 | 23.35 | 6.3 |
| 14.88 | 15.0 | 24.41 | 2.6 |
| 16.18 | 12.4 | 26.49 | 2.8 |
| 17.62 | 23.5 | 27.56 | 2.8 |
| 19.53 | 5.0 | 30.15 | 3.0 |

Preferably, the crystal form A of the meglumine salt has the X-ray powder diffraction intensities shown in Table 10.

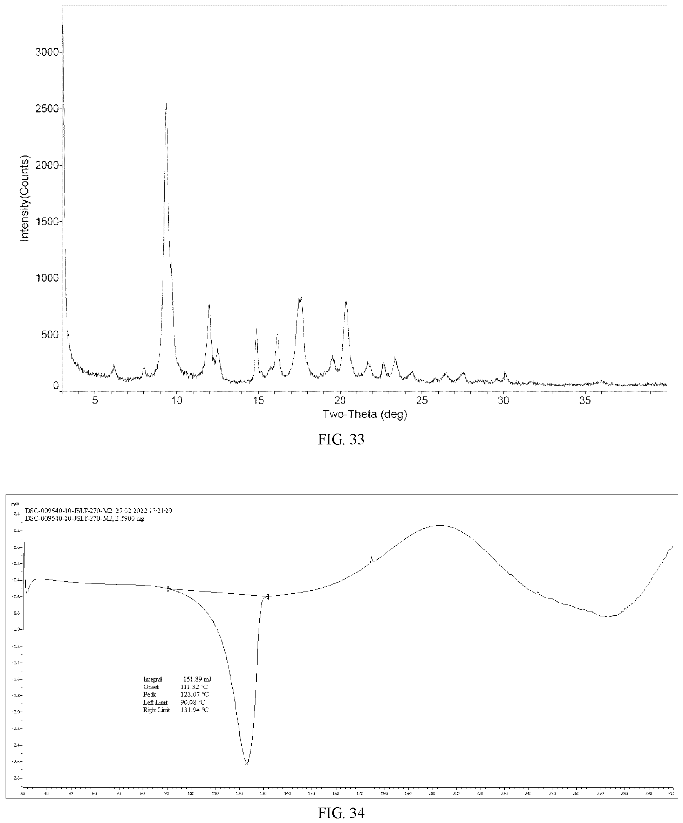

Preferably, the crystal form A of the meglumine salt has an X-ray powder diffraction pattern substantially as shown in FIG. 33.

Preferably, the crystal form A of the meglumine salt has a DSC thermogram with an endothermic peak at a temperature of about 123.07 °C.

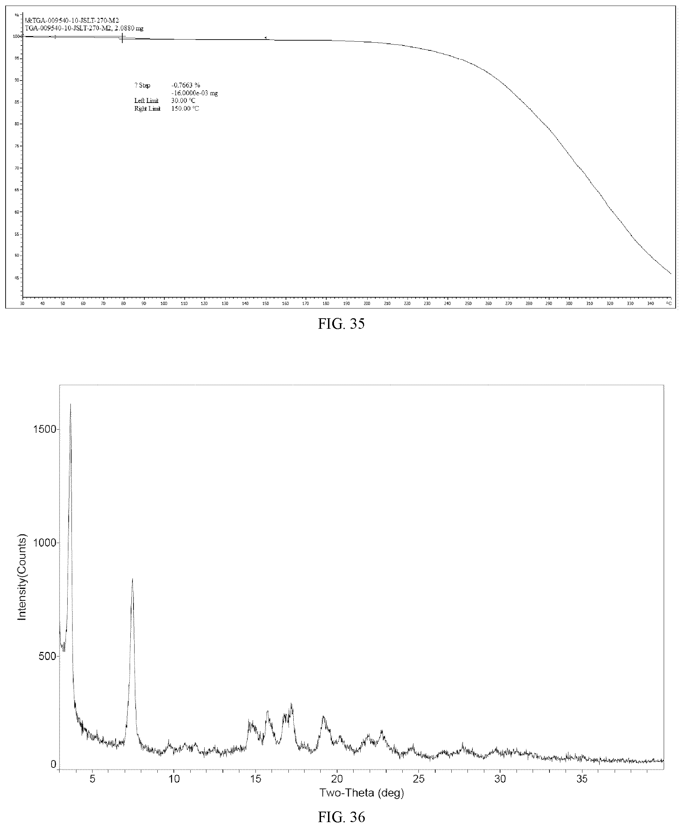

Preferably, the crystal form A of the meglumine salt has a DSC thermogram substantially as shown in FIG. 34. Preferably, the crystal form A of the meglumine salt has a TGA curve substantially as shown in FIG. 35.

As a more preferred embodiment, the present disclosure provides a crystal form A of the tromethamine salt of compound I-2, wherein an X-ray powder diffraction (XRPD) pattern of the crystal form comprises peaks at diffraction angles (20) of 3.68±0.2°, 7.48±0.2°, 17.21±0.2°, and 19.15±0.2°.

As a more preferred embodiment, the present disclosure provides a crystal form A of the tromethamine salt of compound I-2, wherein an X-ray powder diffraction (XRPD) pattern of the crystal form comprises peaks at diffraction angles (20) of 3.68±0.2°, 7.48±0.2°, 17.21±0.2°, 19.15±0.2°, 16.73±0.2°, and 15.74±0.2°.

Preferably, the X-ray powder diffraction pattern of the crystal form A of the tromethamine salt has the diffraction angles (20) shown in Table 11, wherein the 2θ angles have a margin of error of +0.20°:

**Table 11**

| 2θ ( ° ) | Intensity% | 2θ (°) | Intensity% |
|---|---|---|---|
| 3.68 | 100.0 | 19.15 | 9.6 |
| 7.48 | 51.3 | 20.18 | 3.3 |
| 14.82 | 7.2 | 21.95 | 3.9 |
| 15.74 | 9.0 | 22.67 | 4.9 |
| 16.73 | 9.2 | 24.66 | 4.0 |
| 17.21 | 12.7 | 27.68 | 4.3 |

Preferably, the crystal form A of the tromethamine salt has the X-ray powder diffraction intensities shown in Table 11.

Preferably, the crystal form A of the tromethamine salt has an X-ray powder diffraction pattern substantially as shown in FIG. 36.

Preferably, the crystal form A of the tromethamine salt has a DSC thermogram with an endothermic peak at a temperature of about 167.96 °C.

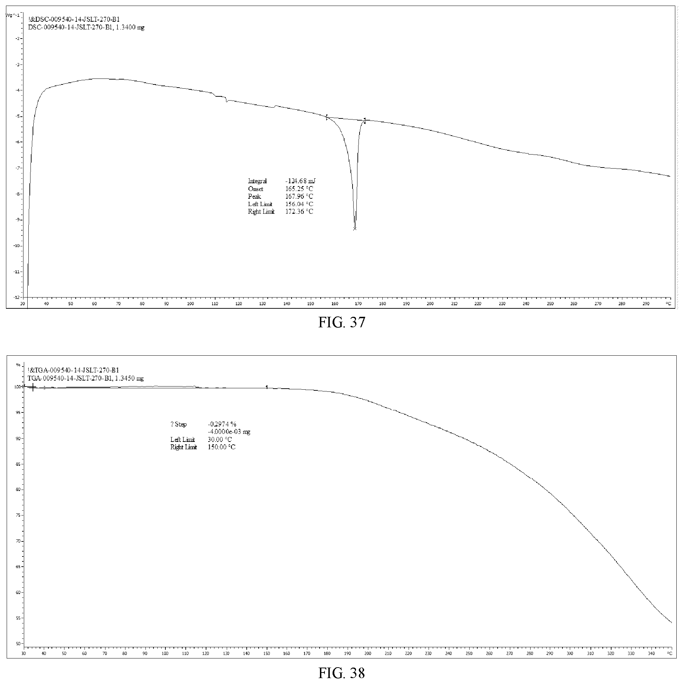

Preferably, the crystal form A of the tromethamine salt has a DSC thermogram substantially as shown in FIG. 37. Preferably, the crystal form A of the tromethamine salt has a TGA curve substantially as shown in FIG. 38.

The present disclosure provides, in a second aspect, a preparation method for a pharmaceutically acceptable salt of compound I-1 or compound I-2, the preparation method comprising reacting compound I-1 or compound I-2 with an acid or base in a solvent to give the pharmaceutically acceptable salt of compound I-1 or compound I-2. According to an embodiment of the present disclosure, the acid is selected from an inorganic acid and an organic acid, wherein the inorganic acid may be selected from hydrobromic acid, hydrochloric acid, sulfuric acid, sulfurous acid, phosphoric acid, and boric acid; the organic acid may be selected from acetic acid, oxalic acid, valeric acid, benzoic acid, lactic acid, toluic acid, citric acid, malic acid, maleic acid, fumaric acid, succinic acid, tartaric acid, methanesulfonic acid, benzenesulfonic acid, and *p*-toluenesulfonic acid.

According to an embodiment of the present disclosure, the base is selected from an inorganic base and an organic base, wherein the inorganic base may be selected from alkali metal hydroxides and alkaline earth metal hydroxides, such as sodium hydroxide, lithium hydroxide, potassium hydroxide, calcium hydroxide, and magnesium hydroxide; the organic base may be selected from ammonia (NH₃), primary amines, secondary amines, and tertiary amines, such as tetramethylamine, tetraethylamine, methylamine, dimethylamine, trimethylamine, triethylamine, ethylamine, meglumine, choline, and tromethamine.

According to an embodiment of the present disclosure, the preparation method further comprises a step of, after the reaction is complete, creating a degree of supersaturation to precipitate a product, wherein the method for creating the degree of supersaturation comprises one or more of the following: adding a crystal seed, volatilizing the solvent, adding an antisolvent, or cooling to give an acid salt or base salt of compound I.

According to an embodiment of the present disclosure, the solvent may be selected from organic solvents of alcohols, chloroalkanes, ketones, ethers, cyclic ethers, esters, alkanes, cycloalkanes, benzenes, amides, sulfoxides, and nitriles, combinations of two or more of the solvents, and mixtures of the above solvents or combinations with water.

According to an embodiment of the present disclosure, the ketones may be selected from ketones having 3-10 carbon atoms, such as acetone, butanone, pentanone, methyl ethyl ketone, methyl isobutyl ketone, and 4-methyl-2-pentanone, and combinations thereof; the nitriles may be selected from acetonitrile; the alcohols may be selected from alcohols and halogenated alcohols having 1-8 carbon atoms, such as methanol, ethanol, n-propanol, isopropanol, n-butanol, neopentanol, and trifluoroethanol, and combinations thereof; the esters may be selected from organic formates, such as methyl formate, ethyl acetate, isobutyl formate, and isopropyl acetate, and combinations thereof; the ethers may be straight or branched alkyl ether or cyclic ether compounds, such as methyl *tert*-butyl ether, tetrahydrofuran, 2-methyl-tetrahydrofuran, or combinations thereof; the chloroalkanes may be selected from dichloromethane, trichloromethane, and 1,2-dichloroethane.

According to an embodiment of the present disclosure, the solvent is selected from methanol, ethanol, n-propanol, isopropanol, acetonitrile, acetone, methyl isobutyl ketone, 1,4-dioxane, tetrahydrofuran, *N,N-*dimethylformamide, ethyl acetate, isopropyl acetate, methyl *tert*-butyl ether, 2-methoxyethyl ether, acetonitrile, water, and mixtures thereof.

According to an embodiment of the present disclosure, a molar ratio of compound I-1 or I-2 to the acid or base may be 1:0.8-1:2, preferably 1:0.9-1:1.8, and more preferably 1:1.0-1:1.5.

According to an embodiment of the present disclosure, in the preparation method, the reaction temperature may be selected within a relatively wide range, e.g., 20 °C-80 °C, preferably 25 °C-60 °C.

According to an embodiment of the present disclosure, the preparation method further comprises a step of, after the reaction is complete, filtering and/or drying to give the pharmaceutically acceptable salt of compound I-1 or compound I-2.

According to an embodiment of the present disclosure, in the preparation method, the drying temperature may be selected within a relatively wide range, e.g., 20 °C-80 °C, preferably 30 °C-60 °C.

The present disclosure further provides preparation methods for a pharmaceutically acceptable salt of compound I-1:
method 1a, comprising: dissolving compound I-1 in acetonitrile, adding concentrated hydrochloric acid, L-tartaric acid, maleic acid, or methanesulfonic acid, stirring at room temperature, then filtering, and drying to give the hydrochloride of compound I-1, the tartrate of compound I-1, the maleate of compound I-1, or the methanesulfonate of compound I-1;
method 1b, comprising: dissolving compound I-1 and citric acid in acetone, stirring at room temperature, then filtering, and drying to give the citrate of compound I-1;
method 1c, comprising: dissolving compound I-1 and sodium hydroxide or potassium hydroxide in acetonitrile or methyl isobutyl ketone, stirring at room temperature, then filtering, and drying to give the sodium salt of compound I-1 or the potassium salt of compound I-1;
method 1d, comprising: dissolving compound I-1 and meglumine in acetonitrile, stirring at room temperature, then filtering, and drying to give the meglumine salt of compound I-1; and
method 1e, comprising: dissolving compound I-1 and tromethamine in N-methylpyrrolidone, adding to toluene, stirring at room temperature, then filtering, and drying to give the tromethamine salt of compound I-1, wherein preferably, a volume ratio of *N*-methylpyrrolidone to toluene is 2:15.

The present disclosure further provides preparation methods for a pharmaceutically acceptable salt of compound I-2:
method 2a, comprising: dissolving compound I-2 and citric acid or L-tartaric acid in acetone, stirring at room temperature, then filtering, and drying to give the citrate of compound I-2 or the tartrate of compound I-2;
method 2b, comprising: dissolving compound I-2 and L-malic acid or fumaric acid in acetonitrile/water, stirring at room temperature, then filtering, and drying to give the malate of compound I-2 or the fumarate of compound I-2, wherein preferably, a volume ratio of acetonitrile to water is 1:1;
method 2c, comprising: dissolving compound I-2 and methanesulfonic acid or maleic acid in ethyl acetate, stirring at room temperature, then filtering, and drying to give the methanesulfonate of compound I-2 or the maleate of compound I-2;
method 2d, comprising: dissolving compound I-2 and sodium hydroxide, potassium hydroxide, calcium hydroxide, or magnesium hydroxide in a mixed solvent of acetonitrile and water or in ethyl acetate, stirring at room temperature, then filtering, and drying to give the sodium salt of compound I-2, the potassium salt of compound I-2, the calcium salt of compound I-2, and the magnesium salt of compound I-2, wherein preferably, a volume ratio of acetonitrile to water is 1:1;
method 2e, comprising: dissolving compound I-2 and meglumine in acetone, stirring at room temperature, then filtering, and drying to give the meglumine salt of compound I-2; and
method 2f, comprising: dissolving compound I-2 and tromethamine in isopropanol, stirring at room temperature, then filtering, and drying to give the tromethamine salt of compound I-2.

The present disclosure provides, in a third aspect, a pharmaceutical composition comprising at least one of the aforementioned pharmaceutically acceptable salts of the compound represented by formula (I) and a pharmaceutically acceptable carrier.

The present disclosure provides, in a fourth aspect, use of at least one of the aforementioned pharmaceutically acceptable salts of the compound represented by formula (I) for the manufacturing of a medicament for the treatment of a metabolic disease, a tumor, an autoimmune disease, or a metastatic disease.

The present disclosure provides, in a fifth aspect, the aforementioned pharmaceutically acceptable salt of the compound represented by formula (I) for use as a medicament for the treatment of a metabolic disease, a tumor, an autoimmune disease, or a metastatic disease.

The present disclosure provides, in a sixth aspect, the aforementioned pharmaceutically acceptable salt of the compound represented by formula (I) for use as a medicament for the prevention or treatment of T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, kidney disease, diabetic retinopathy, adipocyte dysfunction, visceral adipocyte accumulation, sleep apnea, obesity, eating disorders, weight gain caused by use of other medicaments, excessive sugar craving, dyslipidemia, hyperinsulinemia, NAFLD, NAS, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome XI, premenstrual syndrome, angina pectoris, thrombosis, atherosclerosis, transient ischemic attacks, vascular restenosis, impaired glucose metabolism, impaired fasting blood glucose conditions, hyperuricemia, gout, erectile dysfunction, skin and connective tissue abnormalities, psoriasis, foot ulcers, ulcerative colitis, hyperapoB lipoproteinemia, Alzheimer's disease, schizophrenia, impaired cognitive function, inflammatory bowel disease, short bowel syndrome, Crohn's disease, colitis, irritable bowel syndrome, and polycystic ovary syndrome and the treatment of addiction.

As a preferred embodiment, the aforementioned pharmaceutically acceptable salt of the compound represented by formula (I) is used as a medicament for the treatment of T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, obesity, eating disorders, weight gain caused by use of other medicaments, excessive sugar craving, dyslipidemia, and hyperinsulinemia.

The present disclosure further provides a method of treatment of a disease, comprising administering to an individual in need thereof a therapeutically effective amount of at least one of the pharmaceutically acceptable salt of the compound represented by formula (I) described above or the pharmaceutical composition.

According to an embodiment of the present disclosure, the disease is selected from metabolic diseases, tumors, autoimmune diseases, and metastatic diseases.

According to an embodiment of the present disclosure, the disease is selected from T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, kidney disease, diabetic retinopathy, adipocyte dysfunction, visceral adipocyte accumulation, sleep apnea, obesity, eating disorders, weight gain caused by use of other medicaments, excessive sugar craving, dyslipidemia, hyperinsulinemia, NAFLD, NAS, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome XI, premenstrual syndrome, angina pectoris, thrombosis, atherosclerosis, transient ischemic attacks, vascular restenosis, impaired glucose metabolism, impaired fasting blood glucose conditions, hyperuricemia, gout, erectile dysfunction, skin and connective tissue abnormalities, psoriasis, foot ulcers, ulcerative colitis, hyperapoB lipoproteinemia, Alzheimer's disease, schizophrenia, impaired cognitive function, inflammatory bowel disease, short bowel syndrome, Crohn's disease, colitis, irritable bowel syndrome, and polycystic ovary syndrome.

### Beneficial Effects

The inventors have studied a variety of acid or base salts of compound I. The salt form greatly improves the physicochemical properties of compound I such as solubility, hygroscopicity, and chemical stability. The salt-form compounds meet the requirements of industrial production, can meet the needs of the development of clinical pharmaceutical formulations, have very important clinical application value, and are expected to accelerate the development of a new-generation GLP-1R small-molecular agonist.

### BRIEF DESCRIPTION OF THE DRAWINGS

FIG. 1 shows an X-ray powder diffraction pattern of the hydrochloride of compound I-1 of the present disclosure. The abscissa represents 2θ values (degrees), and the ordinate represents peak intensity.
FIG. 2 shows an X-ray powder diffraction pattern of the tartrate of compound I-1 of the present disclosure. The abscissa represents 2θ values (degrees), and the ordinate represents peak intensity.
FIG. 3 shows an X-ray powder diffraction pattern of the citrate of compound I-1 of the present disclosure. The abscissa represents 2θ values (degrees), and the ordinate represents peak intensity.
FIG. 4 shows a DSC thermogram of the citrate of compound I-1 of the present disclosure. The abscissa represents temperature (°C), and the ordinate represents heat flow (mW).
FIG. 5 shows a TGA curve of the citrate of compound I-1 of the present disclosure. The abscissa represents temperature (°C), and the ordinate represents weight (%).
FIG. 6 shows an X-ray powder diffraction pattern of the maleate of compound I-1 of the present disclosure. The abscissa represents 2θ values (degrees), and the ordinate represents peak intensity.
FIG. 7 shows an X-ray powder diffraction pattern of the methanesulfonate of compound I-1 of the present disclosure. The abscissa represents 2θ values (degrees), and the ordinate represents peak intensity.
FIG. 8 shows an X-ray powder diffraction pattern of the sodium salt of compound I-1 of the present disclosure. The abscissa represents 2θ values (degrees), and the ordinate represents peak intensity.
FIG. 9 shows a DSC thermogram of the sodium salt of compound I-1 of the present disclosure. The abscissa represents temperature (°C), and the ordinate represents heat flow (mW).
FIG. 10 shows a TGA curve of the sodium salt of compound I-1 of the present disclosure. The abscissa represents temperature (°C), and the ordinate represents weight (%).
FIG. 11 shows an X-ray powder diffraction pattern of the crystal form A of the potassium salt of compound I-1 of the present disclosure. The abscissa represents 2θ values (degrees), and the ordinate represents peak intensity.
FIG. 12 shows an X-ray powder diffraction pattern of the crystal form B of the potassium salt of compound I-1 of the present disclosure. The abscissa represents 2θ values (degrees), and the ordinate represents peak intensity.
FIG. 13 shows an X-ray powder diffraction pattern of the meglumine salt of compound I-1 of the present disclosure. The abscissa represents 2θ values (degrees), and the ordinate represents peak intensity.
FIG. 14 shows a DSC thermogram of the meglumine salt of compound I-1 of the present disclosure. The abscissa represents temperature (°C), and the ordinate represents heat flow (mW).
FIG. 15 shows a TGA curve of the meglumine salt of compound I-1 of the present disclosure. The abscissa represents temperature (°C), and the ordinate represents weight (%).
FIG. 16 shows an X-ray powder diffraction pattern of the tromethamine salt of compound I-1 of the present disclosure. The abscissa represents 2θ values (degrees), and the ordinate represents peak intensity.
FIG. 17 shows a DSC thermogram of the tromethamine salt of compound I-1 of the present disclosure. The abscissa represents temperature (°C), and the ordinate represents heat flow (mW).
FIG. 18 shows a TGA curve of the tromethamine salt of compound I-1 of the present disclosure. The abscissa represents temperature (°C), and the ordinate represents weight (%).
FIG. 19 shows an X-ray powder diffraction pattern of the citrate of compound I-2 of the present disclosure. The abscissa represents 2θ values (degrees), and the ordinate represents peak intensity.
FIG. 20 shows an X-ray powder diffraction pattern of the malate of compound I-2 of the present disclosure. The abscissa represents 2θ values (degrees), and the ordinate represents peak intensity.
FIG. 21 shows an X-ray powder diffraction pattern of the tartrate of compound I-2 of the present disclosure. The abscissa represents 2θ values (degrees), and the ordinate represents peak intensity.
FIG. 22 shows an X-ray powder diffraction pattern of the fumarate of compound I-2 of the present disclosure. The abscissa represents 2θ values (degrees), and the ordinate represents peak intensity.
FIG. 23 shows an X-ray powder diffraction pattern of the methanesulfonate of compound I-2 of the present disclosure. The abscissa represents 2θ values (degrees), and the ordinate represents peak intensity.
FIG. 24 shows an X-ray powder diffraction pattern of the maleate of compound I-2 of the present disclosure. The abscissa represents 2θ values (degrees), and the ordinate represents peak intensity.
FIG. 25 shows a DSC thermogram of the maleate of compound I-2 of the present disclosure. The abscissa represents temperature (°C), and the ordinate represents heat flow (mW).
FIG. 26 shows a TGA curve of the maleate of compound I-2 of the present disclosure. The abscissa represents temperature (°C), and the ordinate represents weight (%).
FIG. 27 shows an X-ray powder diffraction pattern of the sodium salt of compound I-2 of the present disclosure. The abscissa represents 2θ values (degrees), and the ordinate represents peak intensity.
FIG. 28 shows an X-ray powder diffraction pattern of the potassium salt of compound I-2 of the present disclosure. The abscissa represents 2θ values (degrees), and the ordinate represents peak intensity.
FIG. 29 shows a DSC thermogram of the potassium salt of compound I-2 of the present disclosure. The abscissa represents temperature (°C), and the ordinate represents heat flow (mW).
FIG. 30 shows a TGA curve of the potassium salt of compound I-2 of the present disclosure. The abscissa represents temperature (°C), and the ordinate represents weight (%).
FIG. 31 shows an X-ray powder diffraction pattern of the calcium salt of compound I-2 of the present disclosure. The abscissa represents 2θ values (degrees), and the ordinate represents peak intensity.
FIG. 32 shows an X-ray powder diffraction pattern of the magnesium salt of compound I-2 of the present disclosure. The abscissa represents 2θ values (degrees), and the ordinate represents peak intensity.
FIG. 33 shows an X-ray powder diffraction pattern of the meglumine salt of compound I-2 of the present disclosure. The abscissa represents 2θ values (degrees), and the ordinate represents peak intensity.
FIG. 34 shows a DSC thermogram of the meglumine salt of compound I-2 of the present disclosure. The abscissa represents temperature (°C), and the ordinate represents heat flow (mW).
FIG. 35 shows a TGA curve of the meglumine salt of compound I-2 of the present disclosure. The abscissa represents temperature (°C), and the ordinate represents weight (%).
FIG. 36 shows an X-ray powder diffraction pattern of the tromethamine salt of compound I-2 of the present disclosure. The abscissa represents 2θ values (degrees), and the ordinate represents peak intensity.
FIG. 37 shows a DSC thermogram of the tromethamine salt of compound I-2 of the present disclosure. The abscissa represents temperature (°C), and the ordinate represents heat flow (mW).
FIG. 38 shows a TGA curve of the tromethamine salt of compound I-2 of the present disclosure. The abscissa represents temperature (°C), and the ordinate represents weight (%).
FIG. 39 shows a DVS plot of compound I-1 of the present disclosure. The abscissa represents relative humidity (%), and the ordinate represents weight change (%).
FIG. 40 shows a DVS plot of compound I-2 of the present disclosure. The abscissa represents relative humidity (%), and the ordinate represents weight change (%).
FIG. 41 shows a DVS plot of the tromethamine salt of compound I-1 of the present disclosure. The abscissa represents relative humidity (%), and the ordinate represents weight change (%).
FIG. 42 shows a DVS plot of the tromethamine salt of compound I-2 of the present disclosure. The abscissa represents relative humidity (%), and the ordinate represents weight change (%).

### Definitions and Description

Unless otherwise stated, the following terms used in the specification and the claims have the following meanings. A particular phrase or term, unless otherwise specifically defined, should not be considered uncertain or unclear, but construed according to its common meaning. When referring to a trade name, it is intended to refer to its corresponding commercial product or its active ingredient.

"Pharmaceutical composition" refers to a mixture containing one or more of the compounds described herein or a physiologically/pharmaceutically acceptable salt or prodrug thereof and other chemical components, as well as other components such as physiological/pharmaceutically acceptable carriers and excipients. The purpose of the pharmaceutical composition is to facilitate administration to an organism, promote absorption of the active ingredient, and thereby exert biological activity.

As used herein, "salt" refers to a compound prepared by reacting an organic acid or base drug with a pharmaceutically acceptable inorganic or organic acid or base.

The intermediate compounds of the present disclosure can be prepared by a variety of synthetic methods well known to those skilled in the art, including the specific embodiments listed below, embodiments formed by combinations thereof with other chemical synthetic methods, and equivalent substitutions thereof known to those skilled in the art. Preferred embodiments include but are not limited to the examples of the present disclosure.

The chemical reactions of the specific embodiments of the present disclosure are carried out in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction scheme based on the existing embodiments.

The present disclosure is described in detail below through examples; however, these examples are not intended to limit the present disclosure in any way.

All solvents used in the present disclosure are commercially available and can be used without further purification. Unless otherwise stated, all reactions in the present disclosure were performed under continuous magnetic stirring in dry solvents, and the temperatures are expressed in degrees Celsius (°C).

### Methods and Materials

The structures of the compounds were determined by nuclear magnetic resonance (NMR). The NMR shifts (δ) are given in parts per million (ppm). The NMR analyses were performed using a Bruker avance-400MHz NMR spectrometer with deuterated dimethyl sulfoxide (DMSO-d6) or deuterated methanol (MeOD-d4) as the solvent and tetramethylsilane (TMS) as the internal standard, and the chemical shifts are expressed in 10⁻⁶ ppm.

The HPLC steps were performed using an Agilent 1260 high performance liquid chromatograph or an equivalent high performance liquid chromatograph (with a Sunfire C18 150 × 4.6 m chromatography column or an equivalent chromatography column).

The crystal forms of the acid salts or the base salts of compound I were characterized by X-ray powder diffraction patterns. The X-ray powder diffraction patterns of the salts were collected on a Bruker D8 Advance powder diffractometer operating in reflection mode using Cu Kα radiation. The instrument used Cu Kα radiation (40 kV, 40 mA) and operated at room temperature using an SSD160-2 detector. The 2θ scan range was from 3° to 40°, and the scan speed was 0.1 s/step. The diffraction patterns were analyzed using DIFFRAC.MEA.CENTER software.

XRPD samples were prepared by placing a sample on a monocrystal silicon wafer and pressing the sample powder with a glass slide or an equivalent to ensure that the sample had a flat surface and an appropriate height. The sample holder was then placed into the Bruker D8 Advance instrument, and the X-ray powder diffraction pattern was collected using the instrumental parameters described above. Measurement differences associated with such X-ray powder diffraction analysis results result from a variety of factors including: (a) errors in sample preparation (e.g., sample height), (b) instrument errors, (c) calibration errors, (d) operator errors (including those errors produced when determining the peak positions), and (e) the nature of the material (e.g., preferred orientation errors). Calibration errors and sample height errors often result in a shift of all the peaks in the same direction. In general, this correction factor will bring the measured peak positions into agreement with the expected peak positions and may be in the range of the expected 2θ value ± 0.2°.

The experimental method for characterizing a crystal form of an acid salt or a base salt of compound I by differential scanning calorimetry (DSC) was as follows: A small amount of a powder of the acid salt or the base salt of compound I in the crystal form was taken and placed into an aluminum crucible that matches the instrument and can be sealed with a lid. After the sample was loaded, the crucible was sealed with an aluminum plate and then sent into the instrument for analysis. In this patent, the instrument model used in the differential scanning calorimetry analysis was METTLER TOLEDO DSC 3, and scan parameters were set as follows: a nitrogen atmosphere was used, and the temperature ramp rate was 10.0 k/min.

The experimental method for characterizing a crystal form of an acid salt or a base salt of compound I by thermogravimetric analysis (TGA) was as follows: A small amount of a powder of the acid salt or the base salt of compound I in the crystal form was taken and placed into an aluminum oxide crucible that matches the instrument. After the sample was loaded, the crucible was sent into the instrument for analysis. In the present disclosure, the instrument model used in the differential scanning calorimetry analysis was METTLER TOLEDO TGA 2, and scan parameters were set as follows: a nitrogen atmosphere was used, and the temperature ramp rate was 10.0 k/min. The experimental method for characterizing an acid salt or a base salt of compound I by dynamic vapor sorption (DVS) was as follows: A small amount of a powder of the acid salt or the base salt of compound I was taken and placed into a precise sample tray that matches the instrument. After the sample was loaded, the sample tray was sent into the instrument for analysis. In the present disclosure, the instrument model used in the dynamic vapor sorption analysis was Intrinsic PLUS; the experimental parameter was set as follows: a constant temperature of 25 °C was set; the mass percent change rate per unit time (dm/dt) = 0.02%/min was used as a criterion for determining that a balance is achieved; the program humidity change cycle was set as follows: the initial relative humidity was 0%, and the endpoint relative humidity was 90%.

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented on the basis of the content described above of the present disclosure are encompassed within the claimed scope of the present disclosure.

Unless otherwise stated, the starting materials and reagents used in the following examples are all commercially available products or can be prepared by using known methods.

### Example 1. Preparation of (S)-2-((4-((6-((4-chloro-2-fluorophenoxy)methyl)pyridin-2-yl)oxy)piperidin-1-yl)methyl)-1-(oxetan-2-ylmethy l)-1H-benzo[d]imidazole-6-carboxylic acid (Compound I-1)

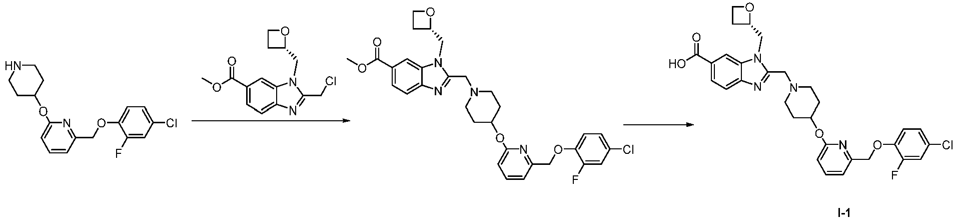

### Step 1: Synthesis of methyl (S)-2-((4-((6-((4-chloro-2-fluorophenoxy)methyl)pyridin-2-yl)oxy)piperidin-1-yl)methyl)-1-(oxetan-2-ylmethyl)-1 H-benzo[d]imidazole-6-carboxylate

A mixed solution of methyl (*S*)-2-(chloromethyl)-1-(oxetan-2-ylmethyl)-1*H*-benzo[*d*]imidazole-6-carboxylate (1.5 g, 5.1 mmol), 2-(4-chloro-2-fluorophenoxy)methyl)-6-(piperidin-4-yloxy)pyridine (1.8 g, 5.5 mmol), and potassium carbonate (1.8 g, 13.0 mmol) in N,N-dimethylformamide (80 mL) was stirred at 60 °C for 3 h, then quenched with water (100 mL), and extracted with ethyl acetate (50 mL × 3). The combined organic layers were washed with brine (50 mL × 3), dried over anhydrous sodium sulfate, filtered, and concentrated. The residue was purified by silica gel column chromatography (dichloromethane/methanol = 20/1) to give methyl (*S*)-2-((4-((6-((4-chloro-2-fluorophenoxy)methyl)pyridin-2-yl)oxy)piperidin-1-yl)methyl)-1-(oxetan-2-ylmethyl)-1 *H*-benzo[*d*]imidazole-6-carboxylate (1.0 g, yield: 33.5%).

### Step 2: Synthesis of (S)-2-((4-((6-((4-chloro-2-fluorophenoxy)methyl)pyridin-2-yl)oxy)piperidin-1-yl)methyl)-1-(oxetan-2-ylmethyl)-1 H-benzo[d]imidazole-6-carboxylic acid

Lithium hydroxide (0.13 g, 5.4 mmol) was added to a mixed solution of methyl (*S*)-2-((4-((6-((4-chloro-2-fluorophenoxy)methyl)pyridin-2-yl)oxy)piperidin-1-yl)methyl)-1-(oxetan-2-ylmethyl)-1 *H*-benzo[*d*]imidazole-6-carboxylate (1.0 g, 1.7 mmol) in tetrahydrofuran/water (20 mL/20 mL), and the mixture was stirred at room temperature for 16 h. The pH of the resulting mixture was adjusted to 5-6 with formic acid, and the solvent was removed *in vacuo.* The residue was purified by reverse-phase flash column chromatography to give (*S*)-2-((4-((6-((4-chloro-2-fluorophenoxy)methyl)pyridin-2-yl)oxy)piperidin-1-yl)methyl)-1-(oxetan-2-ylmethyl)-1 *H*-benzo[*d*]imidazole-6-carboxylic acid (0.69 g, yield: 70.5%). ¹H NMR (400 MHz, DMSO-*d₆*): δ 8.27 (s, 1 H), 7.80 (dd, *J*=8.4 Hz, 1.2 Hz, 1 H), 7.72 (t, *J*=7.6 Hz, 1 H), 7.64 (d, *J*=8.4 Hz, 1 H), 7.44 (dd, *J*=11.2 Hz, 2.0 Hz, 1 H), 7.28 (t, *J*=8.8 Hz, 1 H), 7.18 (d, *J*=8.4 Hz, 1 H), 7.04 (d, *J*=7.2 Hz, 1 H), 6.72 (d, *J*=8.0 Hz, 1 H), 5.18 (s, 2 H), 5.12-5.06 (m, 1 H), 4.95-4.93 (m, 1 H), 4.81-4.76 (m, 1 H), 4.66-4.62 (m, 1 H), 4.51-4.49 (m, 1 H), 4.38-4.36 (m, 1 H), 3.94 (d, *J*=13.6 Hz, 1 H), 3.78 (d, *J*=13.6 Hz, 1 H), 2.79-2.67 (m, 2 H), 2.46-2.41 (m, 1 H), 2.32 (s, 2 H), 1.92-1.91 (m, 2 H), 1.63-1.59 (m, 2 H).

### Example 2. Preparation of (S)-2-((4-((6-((4-cyano-2-fluorophenoxy)methyl)pyridin-2-yl)oxy)piperidin-1-yl)methyl)-1-(oxetan-2-ylmethyl )-1H-benzo[d]imidazole-6-carboxylic acid (Compound I-2)

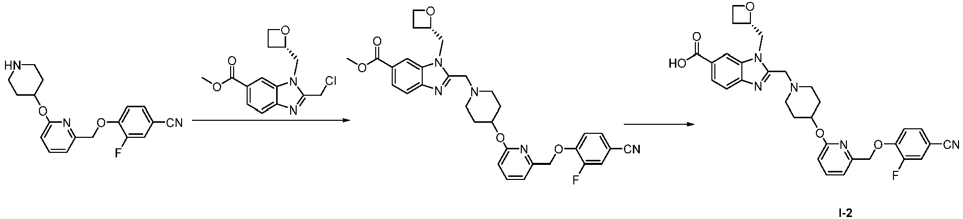

### Step 1: Synthesis of methyl (S)-2-((4-((6-((4-cyano-2-fluorophenoxy)methyl)pyridin-2-yl)oxy)piperidin-1-yl)methyl)-1-(oxetan-2-ylmethyl)-1 H-benzo[d]imidazole-6-carboxylate

A mixed solution of methyl (*S*)-2-(chloromethyl)-1-(oxetan-2-ylmethyl)-1*H*-benzo[*d*]imidazole-6-carboxylate (1.5 g, 5.1 mmol), 2-(4-cyano-2-fluorophenoxy)methyl)-6-(piperidin-4-yloxy)pyridine (1.8 g, 5.5 mmol), and potassium carbonate (1.8 g, 13.0 mmol) in N,N-dimethylformamide (80 mL) was stirred at 60 °C for 3 h, then quenched with water (100 mL), and extracted with ethyl acetate (50 mL × 3). The combined organic layers were washed with brine (50 mL × 3), dried over anhydrous sodium sulfate, filtered, and concentrated. The residue was purified by silica gel column chromatography (dichloromethane/methanol = 20/1) to give methyl (*S*)-2-((4-((6-((4-cyano-2-fluorophenoxy)methyl)pyridin-2-yl)oxy)piperidin-1-yl)methyl)-1-(oxetan-2-ylmethyl)-1 *H*-benzo[*d*]imidazole-6-carboxylate (1.1 g, yield: 37.2%).

### Step 2: Synthesis of (S)-2-((4-((6-((4-cyano-2-fluorophenoxy)methyl)pyridin-2-yl)oxy)piperidin-1-yl)methyl)-1-(oxetan-2-ylmethyl)-1 H-benzo[d]imidazole-6-carboxylic acid

Lithium hydroxide (0.13 g, 5.4 mmol) was added to a mixed solution of methyl (*S*)-2-((4-((6-((4-cyano-2-fluorophenoxy)methyl)pyridin-2-yl)oxy)piperidin-1-yl)methyl)-1-(oxetan-2-ylmethyl)-1 *H*-benzo[*d*]imidazole-6-carboxylate (1.1 g, 1.9 mmol) in tetrahydrofuran/water (20 mL/20 mL), and the mixture was stirred at room temperature for 16 h. The pH of the resulting mixture was adjusted to 5-6 with formic acid, and the solvent was removed *in vacuo.* The residue was purified by reverse-phase flash column chromatography to give (*S*)-2-((4-((6-((4-chloro-2-fluorophenoxy)methyl)pyridin-2-yl)oxy)piperidin-1-yl)methyl)-1-(oxetan-2-ylmethyl)-1 *H*-benzo[*d*]imidazole-6-carboxylic acid (0.70 g, yield: 65.5%). ¹HNMR (400 MHz, DMSO-*d₆*): δ 8.23 (s, 1 H), 7.88 (dd, *J*=2.0 Hz, 11.6 Hz, 1 H), 7.80 (dd, *J =* 1.6, 8.8 Hz, 1 H), 7.73 (t, *J* = 8.0 Hz, 1 H), 7.67 (d, *J* =8.4 Hz ,1 H), 7.60 (*d, J =*8.8 Hz, 1 H), 7.45 (t, *J* = 8.4 Hz, 1 H), 7.06 (d, *J* =7.6 Hz, 1 H), 6.74 (d, J=8.4 Hz, 1 H), 5.31 (s, 2 H), 5.10-5.08 (m, 1 H), 4.92-4.90 (m, 1H), 4.80-4.74 (m, 1 H), 4.65-4.61 (m, 1 H), 4.50-4.47 (m, 1 H), 4.40-4.35 (m, 1 H), 3.93 (d, *J=* 13.6 Hz, 1 H), 3.78 (d, *J =* 13.6 Hz, 1 H), 2.79-2.67 (m, 3 H), 2.51-2.41 (m, 1 H), 2.32-2.27 (m, 2 H), 1.92-1.89 (m, 2 H) 1.63-1.60 (m, 2 H).

### Example 3. Preparation of Hydrochloride of Compound I-1

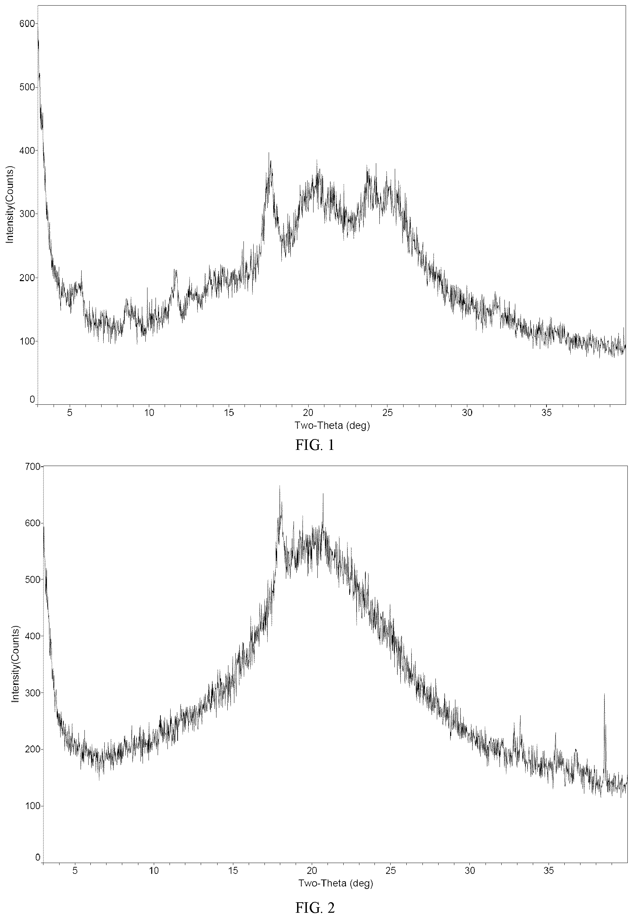

20 mg of compound I-1 was added to 1 mL of acetonitrile, and 10 mg of concentrated hydrochloric acid was added. The mixture was stirred at room temperature for 1 day and filtered, and the filter cake was dried in a drying oven at 50 °C to give the hydrochloride of compound I-1. The product was analyzed and characterized by XRPD (FIG. 1).

### Example 4. Preparation of Tartrate of Compound I-1

20 mg of compound I-1 was added to 1 mL of acetonitrile, and 7.7 mg of L-tartaric acid was added. The mixture was stirred at room temperature for 1 day and filtered, and the filter cake was dried in a drying oven at 50 °C to give the tartrate of compound I-1. The product was analyzed and characterized by XRPD (FIG. 2).

### Example 5. Preparation of Citrate of Compound I-1

199.8 mg of compound I-1 and 66.8 mg of citric acid were added to 5 mL of acetone. The mixture was stirred at room temperature for 1 day and filtered, and the filter cake was dried in a drying oven at 50 °C to give the citrate of compound I-1. Analytical data were collected on the product, including characterization by XRPD (FIG. 3), DSC (FIG. 4), and TGA (FIG. 5). The citrate exhibited a DSC thermogram with endothermic peaks at temperatures of about 107.80 °C and 130.63 °C. ¹H NMR (400 MHz, CD₃OD): δ 8.33 (s, 1 H), 7.98 (dd, J=8.4 Hz, 1.2 Hz, 1 H), 7.71-7.65 (m, 2 H), 7.21-7.05 (m, 4 H), 6.69 (d, *J*=8.0 Hz, 1 H), 5.25-5.23 (1H), 5.13 (s, 3 H), 4.72-4.62 (m, 3 H), 4.48-4.43 (m, 1 H), 4.26-4.13 (m, 2 H), 3.06-3.01 (m, 2 H), 2.89-2.75 (m, 7 H), 2.56-2.49 (m, 1 H), 2.08-2.06 (m, 2 H), 1.89-1.87 (m, 2 H).

### Example 6. Preparation of Maleate of Compound I-1

20 mg of compound I-1 was added to 1 mL of acetonitrile, and 5 mg of maleic acid was added. The mixture was stirred at room temperature for 1 day and filtered, and the filter cake was dried in a drying oven at 50 °C to give the maleate of compound I-1. The product was analyzed and characterized by XRPD (FIG. 6).

### Example 7. Preparation of Methanesulfonate of Compound I-1

20 mg of compound I-1 was added to 1 mL of acetonitrile, and 5.0 mg of methanesulfonic acid was added. The mixture was stirred at room temperature for 1 day and filtered, and the filter cake was dried in a drying oven at 50 °C to give the methanesulfonate of compound I-1. The product was analyzed and characterized by XRPD (FIG. 7).

### Example 8. Preparation of Sodium Salt of Compound I-1

199.8 mg of compound I-1 and 16.5 mg of sodium hydroxide were added to 10 mL of acetonitrile. The mixture was stirred at room temperature for 3 days and filtered, and the filter cake was dried in a drying oven at 50 °C to give the sodium salt of compound I-1. Analytical data were collected on the product, including characterization by XRPD (FIG. 8), DSC (FIG. 9), and TGA (FIG. 10). The sodium salt exhibited a DSC thermogram with endothermic peaks at temperatures of about 149.11 °C and 174.11 °C.

### Example 9. Preparation of Potassium Salt of Compound I-1

20 mg of compound I-1 was added to 0.4 mL of methyl isobutyl ketone, and 2.3 mg of potassium hydroxide was added. The mixture was stirred at room temperature for 3 days and filtered, and the filter cake was dried in a drying oven at 40 °C to give the potassium salt of compound I-1. Analytical data were collected on the product, and the product was analyzed and characterized by XRPD (FIG. 11).

### Example 10. Preparation of Potassium Salt of Compound I-1

20 mg of compound I-1 was added to 0.4 mL of acetonitrile, and 2.3 mg of potassium hydroxide was added. The mixture was stirred at room temperature for 16 h and filtered, and the filter cake was dried in a drying oven at 40 °C to give the potassium salt of compound I-1. Analytical data were collected on the product, and the product was analyzed and characterized by XRPD (FIG. 12).

### Example 11. Preparation of Meglumine Salt of Compound I-1

199.9 mg of compound I-1 and 67.5 mg of meglumine were added to 10 mL of acetonitrile. The mixture was stirred at room temperature for 3 days and filtered, and the filter cake was dried in a drying oven at 40 °C to give the meglumine salt of compound I-1. Analytical data were collected on the product, including characterization by XRPD (FIG. 13), DSC (FIG. 14), and TGA (FIG. 15). The meglumine salt exhibited a DSC thermogram with an endothermic peak at a temperature of about 120.06 °C. ¹H NMR (400 MHz, CD₃OD): δ 8.19 (s, 1 H), 7.94 (dd, *J=8.4* Hz, 1.2 Hz, 1 H), 7.67-7.57 (m, 2 H), 7.20-7.03 (m, 4 H), 6.66 (d, *J*=8.0 Hz, 1 H), 5.28-5.26 (1H), 5.13 (s, 2 H), 5.05-5.04 (m, 1 H), 4.90-4.86 (m, 2 H), 4.73-4.62 (m, 2 H), 4.47-4.45 (m, 1 H), 4.04-3.64 (m, 9 H), 3.14-3.12 (m, 2 H), 2.81-2.77 (m, 3 H), 2.68 (s, 3 H), 2.52-41 (m, 3 H), 2.00-1.98 (m, 2 H), 1.78-1.76 (m, 2 H).

### Example 12. Preparation of Tromethamine Salt of Compound I-1

200 mg of compound I-1 and 42 mg of tromethamine were added to 2.0 mL of N-methylpyrrolidone, and the solution was added dropwise to 15 mL of toluene. The mixture was stirred at room temperature for 16 h and filtered, and the filter cake was dried in a drying oven at 50 °C to give the tromethamine salt of compound I-1. Analytical data were collected on the product, including characterization by XRPD (FIG. 16), DSC (FIG. 17), and TGA (FIG. 18). The tromethamine salt exhibited a DSC thermogram with endothermic peaks at temperatures of about 109.95 °C and 166.02 °C. ¹H NMR (400 MHz, CD₃OD): δ 8.20 (s, 1 H), 7.94 (dd, J=8.4 Hz, 1.2 Hz, 1 H), 7.66-7.57 (m, 2 H), 7.21-7.03 (m, 4 H), 6.66 (d, *J*=8.0 Hz, 1 H), 5.28-5.25 (1H), 5.12 (s, 2 H), 5.05-5.03 (m, 1 H), 4.73-4.62 (m, 2 H), 4.47-4.45 (m, 1 H), 4.02-3.88 (m, 2 H), 3.65 (s, 6 H), 3.44 (d, J=7.2 Hz, 3.5 H), 2.82-2.77 (m, 8 H), 2.51-2.33 (m, 6.5 H), 2.07-1.99 (m, 5.5 H), 1.77-1.76 (m, 2 H).

### Example 13. Preparation of Citrate of Compound I-2

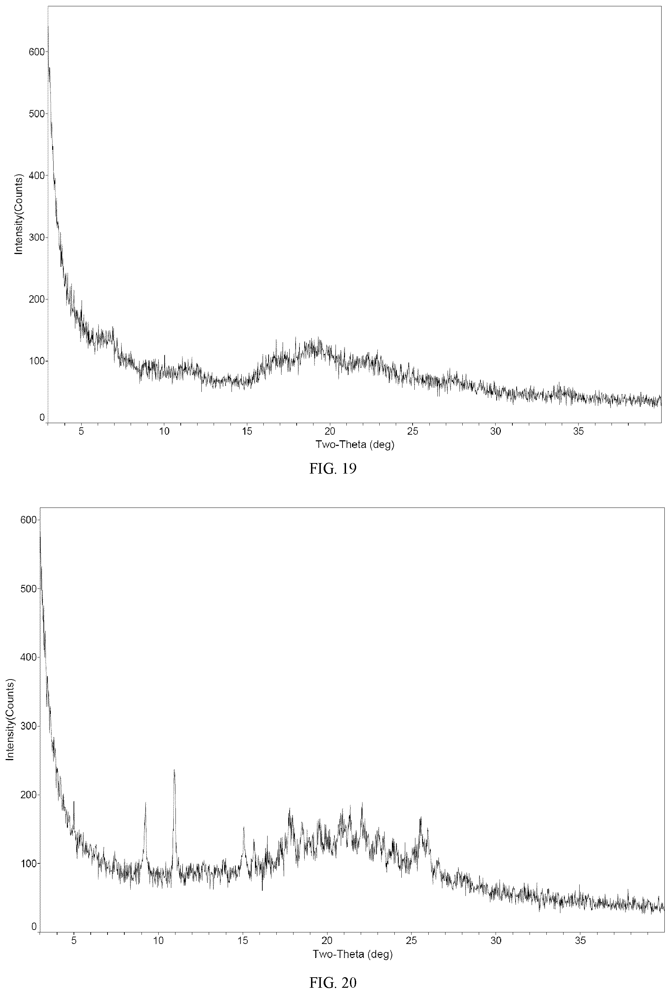

20 mg of compound I-2 and 6.7 mg of citric acid were added to 0.4 mL of acetone. The mixture was stirred at room temperature for 1 day and filtered, and the filter cake was dried in a drying oven at 50 °C to give the citrate of compound I-2. The product was analyzed and characterized by XRPD (FIG. 19).

### Example 14. Preparation of Malate of Compound I-2

20 mg of compound I-2 and 5.6 mg of L-malic acid were added to 0.4 mL of acetonitrile/water (1:1, v:v). The mixture was stirred at room temperature for 3 days and filtered, and the filter cake was dried in a drying oven at 50 °C to give the malate of compound I-2. The product was analyzed and characterized by XRPD (FIG. 20).

### Example 15. Preparation of Tartrate of Compound I-2

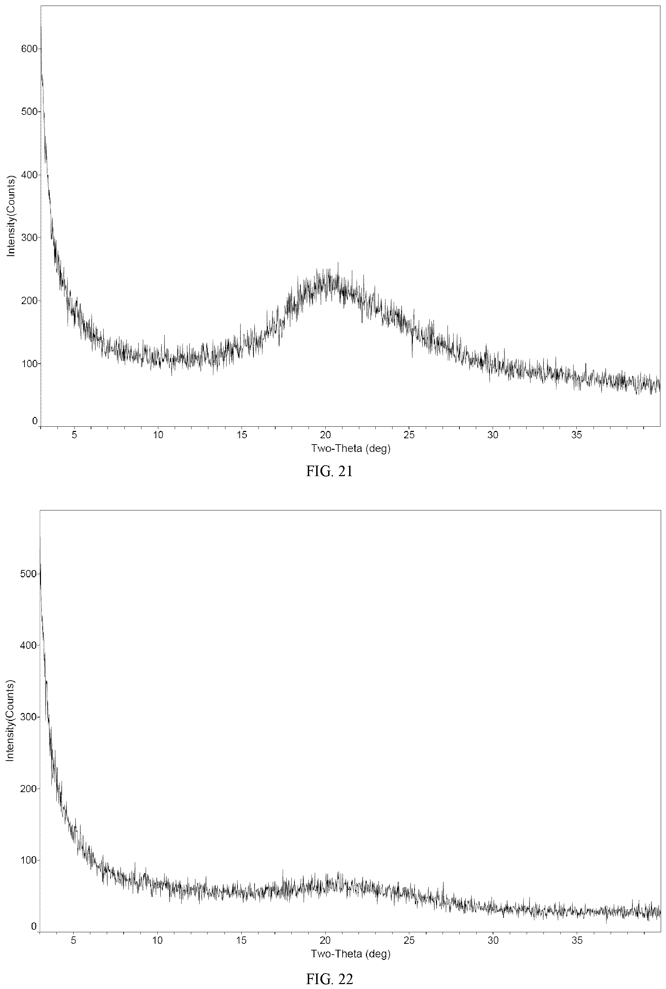

20 mg of compound I-2 and 6.3 mg of L-tartaric acid were added to 0.4 mL of acetone. The mixture was stirred at room temperature for 3 days and filtered, and the filter cake was dried in a drying oven at 50 °C to give the tartrate of compound I-2. The product was analyzed and characterized by XRPD (FIG. 21).

### Example 16. Preparation of Fumarate of Compound I-2

20 mg of compound I-2 and 4.9 mg of fumaric acid were added to 0.4 mL of acetonitrile/water (1:1, v:v). The mixture was stirred at room temperature for 3 days and filtered, and the filter cake was dried in a drying oven at 50 °C to give the fumarate of compound I-2. The product was analyzed and characterized by XRPD (FIG. 22).

### Example 17. Preparation of Methanesulfonate of Compound I-2

20 mg of compound I-2 and 4.0 mg of methanesulfonic acid were added to 0.4 mL of ethyl acetate. The mixture was stirred at room temperature for 3 days and filtered, and the filter cake was dried in a drying oven at 50 °C to give the methanesulfonate of compound I-2. The product was analyzed and characterized by XRPD (FIG. 23).

### Example 18. Preparation of Maleate of Compound I-2

200.1 mg of compound I-2 and 48.6 mg of maleic acid were added to 5 mL of ethyl acetate. The mixture was stirred at room temperature for 3 days and filtered, and the filter cake was dried in a drying oven at 50 °C to give the maleate of compound I-2. Analytical data were collected on the product, including characterization by XRPD (FIG. 24), DSC (FIG. 25), and TGA (FIG. 26). The maleate exhibited a DSC thermogram with an endothermic peak at a temperature of about 119.30 °C. ¹HNMR (400 MHz, CD₃OD): δ 8.34 (s, 1 H), 8.02 (d, *J =* 2.2, 8.6 Hz, 1 H), 7.79-7.71 (m, 2 H), 7.58-7.50 (m, 2 H), 7.33-7.31 (m, 1 H), 7.12 (d, J=7.6 Hz, 1 H), 6.78 (d*, J* =8.5 Hz, 1 H), 6.26 (s, 2H), 5.28-5.24 (m, 4 H), 4.86-4.67 (m, 5 H), 4.45-4.42 (m, 1 H), 3.52-3.45 (m, 4H), 2.90-2.88 (m, 1 H), 2.51-2.49 (m, 1 H), 2.25-2.05 (m, 4 H).

### Example 19. Preparation of Sodium Salt of Compound I-2

20 mg of compound I-2 and 2.8 mg of sodium hydroxide were added to 0.4 mL of acetonitrile/water (1:1, v:v). The mixture was stirred at room temperature for 3 days and filtered, and the filter cake was dried in a drying oven at 50 °C to give the sodium salt of compound I-2. The product was analyzed and characterized by XRPD (FIG. 27).

### Example 20. Preparation of Potassium Salt of Compound I-2

199.8 mg of compound I-2 and 23.6 mg of potassium hydroxide were added to 5 mL of ethyl acetate. The mixture was stirred at room temperature for 3 days and filtered, and the filter cake was dried in a drying oven at 50 °C to give the potassium salt of compound I-2. Analytical data were collected on the product, including characterization by XRPD (FIG. 28), DSC (FIG. 29), and TGA (FIG. 30). The potassium salt exhibited a DSC thermogram with an endothermic peak at a temperature of about 118.44 °C. ¹HNMR (400 MHz, CD₃OD): δ 8.21 (s, 1 H), 7.94 (d, *J =* 8.6 Hz, 1 H), 7.65 (dd, *J* = 1.6*,* 8.5 Hz, 1 H), 7.64-7.55 (m, 2 H), 7.51-7.49 (m, 1 H), 7.34-7.30 (m, 1 H), 7.04 (d, *J* =7.6 Hz, 1 H), 6.68 (d, J=8.5 Hz, 1 H), 5.26-5.24 (m, 3 H), 5.10-5.08 (m, 1 H), 4.90-4.88 (m, 1 H), 4.73-4.62 (m, 2 H), 4.46-4.44 (m, 1 H), 4.02-3.92 (m, 2 H), 2.81-2.77 (m, 3 H), 2.50-2.40 (m, 3 H), 1.92-1.89 (m, 2 H), 1.78-1.75 (m, 2 H).

### Example 21. Preparation of Calcium Salt of Compound I-2

20 mg of compound **I-2** and 5.2 mg of calcium hydroxide were added to 0.4 mL of acetonitrile/water (1:1, v:v). The mixture was stirred at room temperature for 3 days and filtered, and the filter cake was dried in a drying oven at 50 °C to give the calcium salt of compound I-2. The product was analyzed and characterized by XRPD (FIG. 31).

### Example 22. Preparation of Magnesium Salt of Compound I-2

199.8 mg of compound I-2 and 22.4 mg of magnesium hydroxide were added to 5 mL of acetonitrile/water (1:1, v:v). The mixture was stirred at room temperature for 3 days and filtered, and the filter cake was dried in a drying oven at 50 °C to give the magnesium salt of compound I-2. The product was analyzed and characterized by XRPD (FIG. 32).

### Example 23. Preparation of Meglumine Salt of Compound I-2

200.0 mg of compound I-2 and 82.0 mg of meglumine were added to 5 mL of acetone. The mixture was stirred at room temperature for 3 days and filtered, and the filter cake was dried in a drying oven at 50 °C to give the meglumine salt of compound I-2. Analytical data were collected on the product, including characterization by XRPD (FIG. 33), DSC (FIG. 34), and TGA (FIG. 35). The meglumine salt exhibited a DSC thermogram with an endothermic peak at a temperature of about 123.07 °C. ¹HNMR (400 MHz, CD₃OD): δ 8.19 (s, 1 H), 7.94 (dd, *J =* 2.0 Hz, 8.6 Hz, 1 H), 7.66 (dd, *J* = 1.6*,* 8.5 Hz, 1 H), 7.64-7.55 (m, 2 H), 7.51-7.49 (m, 1 H), 7.32 (t, *J=* 8.5 Hz, 1 H), 7.04 (d, *J=* 7.6 Hz, 1 H), 6.68 (d, *J=* 8.5 Hz, 1 H), 5.27-5.24 (m, 3 H), 5.10-5.08 (m, 1 H), 4.90-4.85 (m, 1 H), 4.72-4.62 (m, 2 H), 4.47-4.45 (m, 1 H), 4.03-4.01 (m, 2 H), 3.98-3.92 (m, 1 H), 3.89-3.81 (m, 2 H), 3.79-3.62 (m, 3 H), 3.13-3.11 (m, 2 H), 2.80-2.77 (m, 3 H), 2.68 (s, 3H), 2.51-2.40 (m, 3 H), 2.00-1.98 (m, 2 H), 1.78-1.76 (m, 2 H).

### Example 24. Preparation of Tromethamine Salt of Compound I-2

20 mg of compound I-2 and 4.2 mg of tromethamine were added to 0.5 mL of isopropanol. The mixture was stirred at room temperature for 3 days and filtered, and the filter cake was dried in a drying oven at 50 °C to give the tromethamine salt of compound I-2. Analytical data were collected on the product, including characterization by XRPD (FIG. 36), DSC (FIG. 37), and TGA (FIG. 38). The tromethamine salt exhibited a DSC thermogram with an endothermic peak at a temperature of about 167.96 °C. ¹HNMR (400 MHz, CD₃OD): δ 8.20 (s, 1 H), 7.94 (dd, *J =* 2.0 Hz, 8.6 Hz, 1 H), 7.66 (dd, *J =* 1.6, 8.5 Hz, 1 H), 7.64-7.54 (m, 2 H), 7.51-7.50 (m, 1 H), 7.32 (t, *J=* 8.5 Hz, 1 H), 7.04 (d, *J=* 7.6 Hz, 1 H), 6.68 (d, *J =*8.5 Hz, 1 H), 5.27-5.24 (m, 3 H), 5.10-5.08 (m, 1 H), 4.90-4.88 (m, 1 H), 4.73-4.62 (m, 2 H), 4.47-4.45 (m, 1 H), 4.00 (d, *J* = 13.5 Hz, 1 H), 3.90 (d, *J* = 13.5 Hz, 1 H), 3.65 (s, 6 H), 2.81-2.77 (m, 3 H), 2.51-2.41 (m, 3 H), 1.91-1.89 (m, 2 H), 1.79-1.76 (m, 2 H).

### Example 25. Solubility Study of Salt Forms of Compounds in Water

Free form of compound I-1 and compound I-2, as well as the corresponding representative tromethamine salts, were tested for equilibrium solubility in water (H₂O). In the tests, the solids were suspended in corresponding buffers to formulate suspensions (~10 mg/mL), and the suspensions were stirred at 37±2 °C. After 24 h, samples of the suspensions were taken. The supernatant was filtered, and the concentration was determined. The test results are shown in the table below:

| Solid form | Preparation method | Solution | Equilibrium solubility |
|---|---|---|---|
| Free form of compound I-1 | Example 1 | H₂O | 0.19 mg/mL |
| Tromethamine salt of compound I-1 | Example 12 | H₂O | 0.44 mg/mL |
| Sodium salt of compound I-1 | Example 8 | H₂O | 5.00 mg/mL |
| Potassium salt of compound I-1 | Example 10 | H₂O | 3.33 mg/mL |
| Meglumine salt of compound I-1 | Example 11 | H₂O | 4.00 mg/mL |
| Free form of compound I-2 | Example 2 | H₂O | 0.09 mg/mL |
| Tromethamine salt of compound I-2 | Example 24 | H₂O | 0.33 mg/mL |
| Maleate of compound I-2 | Example 18 | H₂O | 0.11 mg/mL |
| Potassium salt of compound I-2 | Example 20 | H₂O | 0.11 mg/mL |
| Magnesium salt of compound I-2 | Example 22 | H₂O | 0.13 mg/mL |

From the above experimental results, it can be seen that the solubility of the majority of the representative salt forms of compound I-1 of the present disclosure, such as the tromethamine salt, the sodium salt, the potassium salt, the meglumine salt, etc., in water (H₂O) was significantly (several times to several dozen times) higher than that of the free form.

### Example 26. Solubility Study of Tromethamine Salts of Compounds in Other Media

Free form of compound I-1 and compound I-2, as well as the corresponding representative tromethamine salts, were tested for equilibrium solubility in fasted state simulated gastric fluid (FaSSGF), fasted state simulated intestinal fluid (FaSSIF), and fed state simulated intestinal fluid (FeSSIF). In the tests, the solids were suspended in corresponding buffers to formulate suspensions (~10 mg/mL), and the suspensions were stirred at 37±2 °C. After 24 h, samples of the suspensions were taken. The supernatant was filtered, and the concentration was determined. The test results are shown in the table below:

| Solid form | Preparation method | Solution | Equilibrium solubility |
|---|---|---|---|
| Free form of compound I-1 | Example 1 | FaSSIF | 0.05 mg/mL |
| | | FeSSIF | 0.16 mg/mL |
| Tromethamine salt of compound I-1 | Example 12 | FaSSIF | 0.11 mg/mL |
| | | FeSSIF | 0.45 mg/mL |
| Free form of compound I-2 | Example 2 | FaSSGF | 0.18 mg/mL |
| | | FaSSIF | 0.04 mg/mL |
| Tromethamine salt of compound I-2 | Example 24 | FaSSGF | 0.32 mg/mL |
| | | FaSSIF | 0.11 mg/mL |

From the above experimental results, it can be seen that the solubility of the representative tromethamine salts of compound I-1 and compound I-2 in fasted state simulated gastric fluid (FaSSGF), fasted state simulated intestinal fluid (FaSSIF), and fed state simulated intestinal fluid (FeSSIF) was significantly better than that of the free form of compounds.

### Example 27. Hygroscopic Behavior Tests

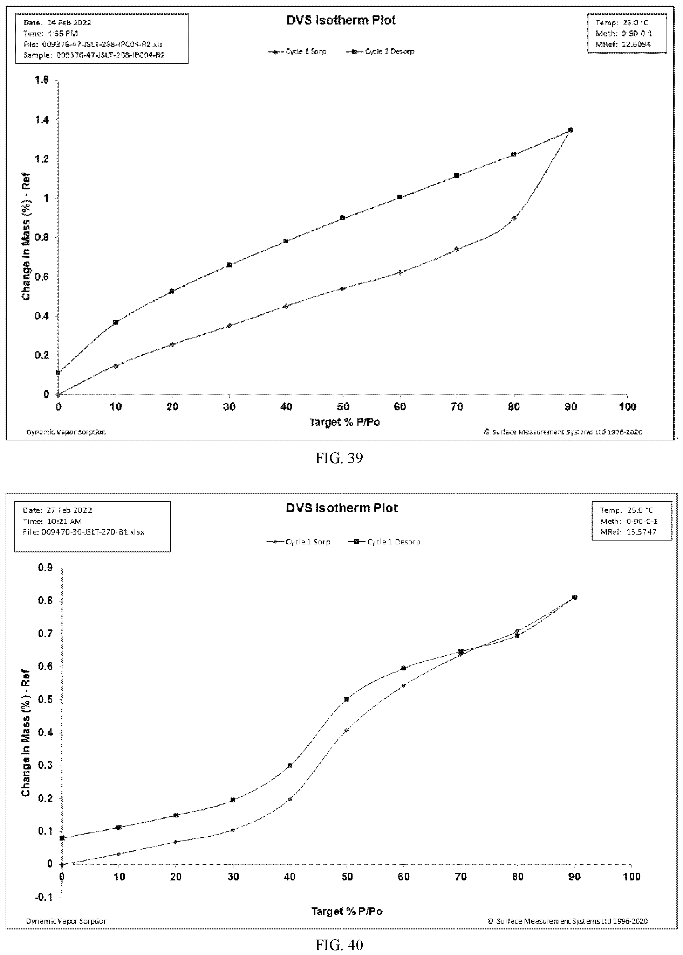

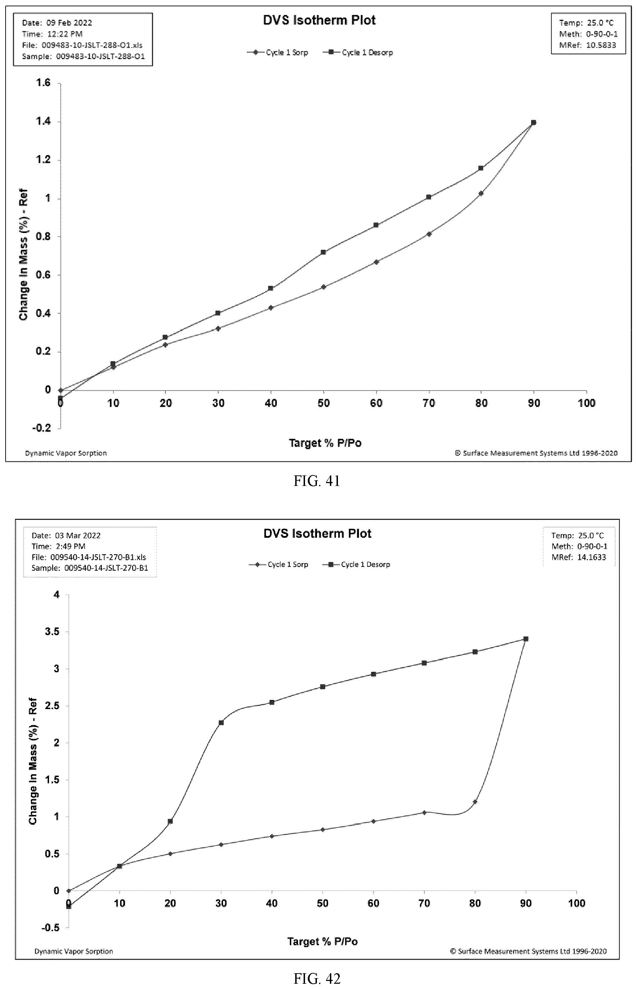

The hygroscopic behavior of a compound affects the production, storage, stability, and quality of the drug. The inventors assessed the stability risk of samples under varying humidity at 25°C using the dynamic vapor sorption method and conducted DVS tests on the representative salt forms of compound I-1 and compound I-2, the tromethamine salts, to evaluate the hygroscopicity of the salt forms of the compounds. The DVS plot of free form of compound I-1 is shown in FIG. 39. The DVS plot of free form of compound I-2 is shown in FIG. 40. The DVS plot of the tromethamine salt of compound I-1 is shown in FIG. 41. The DVS plot of the tromethamine salt of compound I-2 is shown in FIG. 42. The obtained results are shown in the table below:

| Solid form | Preparation method | 80% RH sample weight change (%) | Did the crystal form change before and after the test? | Hygroscopicity |
|---|---|---|---|---|
| Free form of compound I-1 | Example 1 | 0.90 | No | Slightly hygroscopic |
| Tromethamine salt of compound I-1 | Example 12 | 1.03 | No | Slightly hygroscopic |
| Free form of compound I-2 | Example 2 | 0.71 | No | Slightly hygroscopic |
| Tromethamine salt of compound I-2 | Example 24 | 1.20 | No | Slightly hygroscopic |

From the above experimental results, it was unexpectedly found that despite the significant increase in solubility after salt formation, the hygroscopicity did not significantly change compared to the base. On the adsorption curves of 0-90% RH, under the condition of 80% RH, free form of compounds I-1 and I-2 and the tromethamine salts of compounds I-1 and I-2 were all only slightly hygroscopic; there was no significant difference in hygroscopicity, and no solid form change was observed.

The thermal analysis of some of the salt forms of the compounds of the present disclosure is summarized in the table below:

| Salt form | DSC endothermic peak temperature | TGA analysis | Solvent residue |
|---|---|---|---|
| Citrate of compound I-1 | 107.80 °C and 130.63 °C | Weight loss of 2.725% between 30 °C and 135 °C | Acetone |
| Sodium salt of compound I-1 | 149.11 and 174.11 °C | Weight loss of 1.5107% between 30 °C and 160 °C | Acetonitrile |
| Meglumine salt of compound I-1 | 120.06 °C | Weight loss of 5.8772% between 30 °C and 200 °C | Solvent-free |
| Tromethamine salt of compound I-1 | 109.95 °C and 166.02 °C | Weight loss of 18.8996% between 30 °C and 170 °C | *N*-Methylpyrrolidone |
| Maleate of | 119.30 °C | Weight loss of 0.5268% between | Ethyl acetate |

| | | | |
|---|---|---|---|
| compound I-2 | | 30 °C and 130 °C | |
| Potassium salt of compound I-2 | 118.44 °C | Weight loss of 6.5463% between 30 °C and 150 °C | Ethyl acetate |
| Meglumine salt of compound I-2 | 123.07 °C | Weight loss of 0.7663% between 30 °C and 150 °C | Acetone |
| Tromethamine salt of compound I-2 | 167.96 °C | Weight loss of 0.2974% between 30 °C and 150 °C | Isopropanol |

The embodiments of the technical solutions of the present disclosure have been described above by way of example. It should be understood that the protection scope of the present disclosure is not limited to the embodiments described above. Any modification, equivalent substitution, improvement, and the like made by those skilled in the art without departing from the spirit and principle of the present disclosure shall fall within the protection scope of the claims of the present application.

## Claims

1. A pharmaceutically acceptable salt of a compound represented by formula (I), 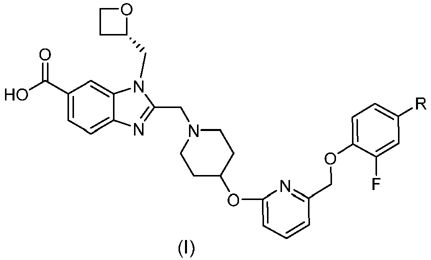
wherein R is selected from halogen and CN;
the pharmaceutically acceptable salt refers to a pharmaceutically non-toxic acid addition salt or base addition salt; preferably, the acid addition salt is a salt formed by the compound represented by formula (I) with an inorganic or organic acid, including a hydrobromide, a hydrochloride, a sulfate, a bisulfate, a sulfite, a phosphate, a borate, an acetate, an oxalate, a valerate, a benzoate, a lactate, a toluate, a citrate, a malate, a maleate, a fumarate, a succinate, a tartrate, a methanesulfonate, a benzenesulfonate, and a p-toluenesulfonate; more preferably, the acid addition salt is a hydrochloride, an acetate, a citrate, a malate, a succinate, a tartrate, a fumarate, a maleate, or a methanesulfonate; particularly, the acid addition salt is a citrate or a maleate;
preferably, the base addition salt is a salt formed by the compound represented by formula (I) with an inorganic or organic base, including salts formed with alkali metals, such as a sodium salt, a lithium salt, a potassium salt, a calcium salt, a magnesium salt, and the like, and amine salts, including salts formed with ammonia (NH₃), primary amines, secondary amines, or tertiary amines, such as a tetramethylamine salt, a tetraethylamine salt, a methylamine salt, a dimethylamine salt, a trimethylamine salt, a triethylamine salt, an ethylamine salt, a meglumine salt, a choline salt, and a tromethamine salt; more preferably, the base addition salt is a sodium salt, a potassium salt, a calcium salt, a magnesium salt, a meglumine salt, a choline salt, or a tromethamine salt; particularly, the base addition salt is a sodium salt, a potassium salt, a magnesium salt, a meglumine salt, or a tromethamine salt;
preferably, the compound represented by formula (I) is selected from the following compound I-1 and compound I-2: 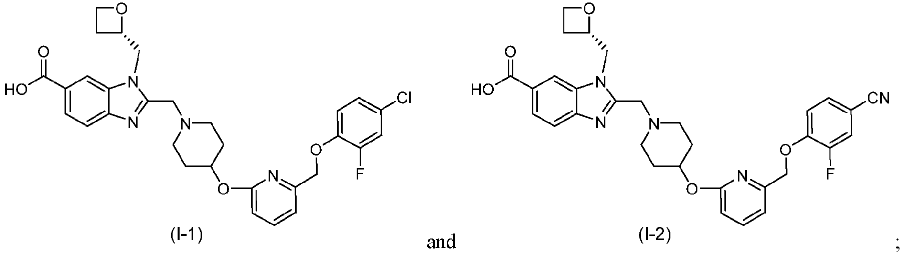
preferably, an acid addition salt of compound I-1 is a hydrochloride, a tartrate, a maleate, a methanesulfonate, or a citrate; an acid addition salt of compound I-2 is a citrate, a tartrate, a malate (e.g., an L-malate), a fumarate, a methanesulfonate, or a maleate;
preferably, a base addition salt of compound I-1 is a sodium salt, a potassium salt, a meglumine salt, or a tromethamine salt; a base addition salt of compound I-2 is a sodium salt, a potassium salt, a calcium salt, a magnesium salt, a meglumine salt, or a tromethamine salt.

2. A crystal form A of the citrate of compound I-1 as claimed in claim 1, wherein an X-ray powder diffraction (XRPD) pattern of the crystal form comprises peaks at diffraction angles (20) of 19.77±0.2°, 16.59±0.2°, 22.47±0.2°, and 20.20±0.2°;
preferably, the X-ray powder diffraction (XRPD) pattern of the crystal form comprises peaks at diffraction angles (20) of 16.59±0.2°, 19.77±0.2°, 22.47±0.2°, 20.20±0.2°, 24.84±0.2°, and 17.51±0.2°;
preferably, the X-ray powder diffraction pattern of the crystal form A of the citrate has the diffraction angles (20) shown in Table 1, wherein the 2θ angles have a margin of error of ±0.20°:
**Table 1**
| 2θ ( ° ) | Intensity% | 2θ(°) | Intensity% |
|---|---|---|---|
| 6.80 | 34.3 | 18.07 | 52.1 |
| 7.13 | 36.4 | 19.77 | 100.0 |
| 8.75 | 33.6 | 20.20 | 61.4 |
| 11.47 | 48.6 | 21.02 | 22.9 |
| 12.06 | 46.4 | 22.47 | 68.6 |
| 13.81 | 27.9 | 24.84 | 60.7 |
| 16.59 | 82.9 | 27.78 | 40.7 |
| 17.51 | 53.6 | | |
preferably, the crystal form A of the citrate has the X-ray powder diffraction intensities shown in Table 1;
preferably, the crystal form A of the citrate has an X-ray powder diffraction pattern substantially as shown in FIG. 3;
preferably, the crystal form A of the citrate has a DSC thermogram with endothermic peaks at temperatures of about 107.80 °C and 130.63 °C;
preferably, the crystal form A of the citrate has a DSC thermogram substantially as shown in FIG. 4;
preferably, the crystal form A of the citrate has a TGA curve substantially as shown in FIG. 5.

3. A crystal form A of the sodium salt of compound I-1 as claimed in claim 1, wherein an X-ray powder diffraction (XRPD) pattern of the crystal form comprises peaks at diffraction angles (2θ) of 19.24±0.2°, 20.68±0.2°, 6.81±0.2°, and 14.43±0.2°;
preferably, the X-ray powder diffraction (XRPD) pattern of the crystal form comprises peaks at diffraction angles (20) of 19.24±0.2°, 20.68±0.2°, 6.81±0.2°, 14.43±0.2°, 14.98±0.2°, and 6.40±0.2°;
preferably, the X-ray powder diffraction pattern of the crystal form A of the sodium salt has the diffraction angles (2θ) shown in Table 2, wherein the 2θ angles have a margin of error of ±0.20°:
**Table 2**
| 2θ ( ° ) | Intensity% | 2θ ( ° ) | Intensity% |
|---|---|---|---|
| 4.07 | 36.4 | 18.02 | 40.9 |
| 6.40 | 47.5 | 19.24 | 100.0 |
| 6.81 | 55.0 | 20.68 | 90.1 |
| 14.43 | 53.3 | 24.27 | 34.3 |
| 14.98 | 52.1 | 24.84 | 29.8 |
| 17.69 | 37.6 | 26.44 | 26.4 |
preferably, the crystal form A of the sodium salt has the X-ray powder diffraction intensities shown in Table 2;
preferably, the crystal form A of the sodium salt has an X-ray powder diffraction pattern substantially as shown in FIG. 8;
preferably, the crystal form A of the sodium salt has a DSC thermogram with endothermic peaks at temperatures of about 149.11 °C and 174.11 °C;
preferably, the crystal form A of the sodium salt has a DSC thermogram substantially as shown in FIG. 9;
preferably, the crystal form A of the sodium salt has a TGA curve substantially as shown in FIG. 10.

4. A crystal form A of the potassium salt of compound I-1 as claimed in claim 1, wherein an X-ray powder diffraction (XRPD) pattern of the crystal form comprises peaks at diffraction angles (2θ) of 13.90±0.2°, 14.43±0.2°, 16.20±0.2°, and 11.67±0.2°;
preferably, the X-ray powder diffraction (XRPD) pattern of the crystal form comprises peaks at diffraction angles (20) of 13.90±0.2°, 14.43±0.2°, 16.20±0.2°, 11.67±0.2°, 20.99±0.2°, and 16.79±0.2°;
preferably, the X-ray powder diffraction pattern of the crystal form A of the potassium salt has the diffraction angles (20) shown in Table 3, wherein the 2θ angles have a margin of error of ±0.20°:
**Table 3**
| 2θ ( ° ) | Intensity% | 2θ ( ° ) | Intensity% |
|---|---|---|---|
| 5.80 | 23.5 | 18.95 | 31.1 |
| 6.60 | 36.0 | 20.39 | 33.9 |
| 8.76 | 4.8 | 20.99 | 47.4 |
| 9.99 | 32.7 | 21.95 | 39.3 |
| 11.67 | 49.1 | 23.06 | 29.2 |
| 12.11 | 21.0 | 23.62 | 39.0 |
| 13.42 | 37.7 | 24.91 | 32.8 |
| 13.90 | 100.0 | 25.45 | 15.2 |
| 14.43 | 96.1 | 26.39 | 21.0 |
| 16.20 | 70.6 | 27.80 | 8.3 |
| 16.79 | 45.8 | 32.76 | 7.2 |
| 17.25 | 13.5 | | |
preferably, the crystal form A of the potassium salt has the X-ray powder diffraction intensities shown in Table 3;
preferably, the crystal form A of the potassium salt has an X-ray powder diffraction pattern substantially as shown in FIG. 11.

5. A crystal form B of the potassium salt of compound I-1 as claimed in claim 1, wherein an X-ray powder diffraction (XRPD) pattern of the crystal form comprises peaks at diffraction angles (20) of 5.92±0.2°, 14.10±0.2°, 17.62±0.2°, and 17.94±0.2°;
preferably, the X-ray powder diffraction (XRPD) pattern of the crystal form comprises peaks at diffraction angles (20) of 5.92±0.2°, 14.10±0.2°, 17.62±0.2°, 17.94±0.2°, 11.92±0.2°, and 7.01±0.2°;
preferably, the X-ray powder diffraction pattern of the crystal form B of the potassium salt has the diffraction angles (20) shown in Table 4, wherein the 2θ angles have a margin of error of ±0.20°:
**Table 4**
| 2θ ( ° ) | Intensity% | 2θ ( ° ) | Intensity% |
|---|---|---|---|
| 5.92 | 100.0 | 20.12 | 11.0 |
| 7.01 | 40.3 | 20.69 | 17.8 |
| 8.74 | 22.1 | 20.90 | 20.8 |
| 9.56 | 11.0 | 21.84 | 39.4 |
| 10.92 | 19.2 | 22.24 | 31.4 |
| 11.92 | 70.3 | 23.33 | 18.4 |
| 12.27 | 17.2 | 24.01 | 23.3 |
| 14.10 | 95.8 | 25.17 | 13.2 |
| 15.19 | 28.1 | 25.69 | 17.4 |
| 15.64 | 19.5 | 26.68 | 15.6 |
| 16.94 | 19.8 | 27.09 | 10.0 |
| 17.62 | 89.6 | 28.38 | 6.7 |
| 17.94 | 80.4 | 29.37 | 8.5 |
| 18.74 | 20.7 | 32.02 | 8.9 |
preferably, the crystal form B of the potassium salt has the X-ray powder diffraction intensities shown in Table 4;
preferably, the crystal form B of the potassium salt has an X-ray powder diffraction pattern substantially as shown in FIG. 12.

6. A crystal form A of the meglumine salt of compound I-1 as claimed in claim 1, wherein an X-ray powder diffraction (XRPD) pattern of the crystal form comprises peaks at diffraction angles (2θ) of 18.15±0.2°, 12.87±0.2°, 22.87±0.2°, and 24.66±0.2°;
preferably, the X-ray powder diffraction (XRPD) pattern of the crystal form comprises peaks at diffraction angles (20) of 18.15±0.2°, 12.87±0.2°, 22.87±0.2°, 24.66±0.2°, 23.21±0.2°, and 19.57±0.2°;
preferably, the X-ray powder diffraction pattern of the crystal form A of the meglumine salt has the diffraction angles (20) shown in Table 5, wherein the 2θ angles have a margin of error of ±0.20°:
**Table 5**
| 2θ ( ° ) | Intensity% | 2θ ( ° ) | Intensity% |
|---|---|---|---|
| 5.221 | 28.9 | 17.26 | 15.5 |
| 7.57 | 9.0 | 18.15 | 100.0 |
| 9.42 | 16.1 | 19.57 | 43.7 |
| 10.23 | 10.0 | 21.39 | 21.9 |
| 10.71 | 20.6 | 22.87 | 55.0 |
| 11.84 | 21.6 | 23.21 | 44.4 |
| 12.87 | 68.1 | 24.66 | 49.5 |
| 15.48 | 16.0 | 25.77 | 13.4 |
preferably, the crystal form A of the meglumine salt has the X-ray powder diffraction intensities shown in Table 5;
preferably, the crystal form A of the meglumine salt has an X-ray powder diffraction pattern substantially as shown in FIG. 13;
preferably, the crystal form A of the meglumine salt has a DSC thermogram with an endothermic peak at a temperature of about 120.06 °C;
preferably, the crystal form A of the meglumine salt has a DSC thermogram substantially as shown in FIG. 14;
preferably, the crystal form A of the meglumine salt has a TGA curve substantially as shown in FIG. 15.

7. A crystal form A of the tromethamine salt of compound I-1 as claimed in claim 1, wherein an X-ray powder diffraction (XRPD) pattern of the crystal form comprises peaks at diffraction angles (2θ) of 3.50±0.2°, 6.97±0.2°, 13.91±0.2°, and 22.19±0.2°;
preferably, the X-ray powder diffraction (XRPD) pattern of the crystal form comprises peaks at diffraction angles (20) of 3.50±0.2°, 6.97±0.2°, 13.91±0.2°, 22.19±0.2°, 31.61±0.2°, 18.11±0.2°, and 20.55±0.2°;
preferably, the crystal form A of the tromethamine salt has the X-ray powder diffraction data shown in Table 6 below:
**Table 6**
| 2θ ( ° ) | Intensity% | 2θ(°) | Intensity% |
|---|---|---|---|
| 3.505 | 100.0 | 20.553 | 14.0 |
| 6.972 | 98.6 | 20.864 | 9.0 |
| 10.168 | 6.1 | 22.190 | 28.8 |
| 11.067 | 5.1 | 23.433 | 2.9 |
| 11.490 | 2.8 | 23.885 | 6.0 |
| 13.910 | 35.2 | 24.275 | 3.5 |
| 14.417 | 5.2 | 26.026 | 8.2 |
| 14.924 | 2.3 | 27.098 | 6.1 |
| 15.762 | 11.9 | 27.481 | 4.0 |
| 16.365 | 8.7 | 27.994 | 5.3 |
| 16.911 | 3.9 | 28.520 | 11.0 |
| 17.185 | 6.5 | 30.271 | 5.1 |
| 17.805 | 4.8 | 31.617 | 18.4 |
| 18.118 | 17.3 | 33.113 | 3.4 |
| 18.974 | 13.9 | 35.165 | 9.7 |
| 19.714 | 3.9 | 38.801 | 2.5 |
preferably, the crystal form A of the tromethamine salt has the X-ray powder diffraction intensities shown in Table 6;
preferably, the crystal form A of the tromethamine salt has an X-ray powder diffraction pattern substantially as shown in FIG. 16;
preferably, the crystal form A of the tromethamine salt has a DSC thermogram with endothermic peaks at temperatures of about 109.95 °C and 166.02 °C;
preferably, the crystal form A of the tromethamine salt has a DSC thermogram substantially as shown in FIG. 17;
preferably, the crystal form A of the tromethamine salt has a TGA curve substantially as shown in FIG. 18;
preferably, the crystal form A of the tromethamine salt is a N-methylpyrrolidone solvate.

8. A crystal form A of the maleate of compound I-2 as claimed in claim 1, wherein an X-ray powder diffraction (XRPD) pattern of the crystal form comprises peaks at diffraction angles (20) of 5.43±0.2°, 9.89±0.2°, 12.76±0.2°, and 8.30±0.2°;
preferably, the X-ray powder diffraction (XRPD) pattern of the crystal form comprises peaks at diffraction angles (20) of 5.43±0.2°, 9.89±0.2°, 12.76±0.2°, 8.30±0.2°, 21.31±0.2°, and 14.24±0.2°;
preferably, the X-ray powder diffraction pattern of the crystal form A of the maleate has the diffraction angles (20) shown in Table 7, wherein the 2θ angles have a margin of error of ±0.20°:
**Table 7**
| 2θ(°) | Intensity% | 2θ(°) | Intensity% |
|---|---|---|---|
| 5.43 | 100.0 | 18.42 | 5.1 |
| 8.30 | 19.9 | 19.16 | 11.9 |
| 9.15 | 5.6 | 19.62 | 10.5 |
| 9.89 | 23.8 | 20.08 | 4.5 |
| 10.94 | 11.9 | 21.31 | 19.3 |
| 12.76 | 22.0 | 22.09 | 9.8 |
| 14.24 | 16.0 | 24.87 | 10.0 |
| 14.84 | 5.3 | 25.32 | 5.5 |
| 16.22 | 6.2 | 25.77 | 8.1 |
| 16.98 | 10.0 | 28.08 | 5.4 |
| 17.43 | 10.9 | 28.40 | 4.1 |
preferably, the crystal form A of the maleate has the X-ray powder diffraction intensities shown in Table 7;
preferably, the crystal form A of the maleate has an X-ray powder diffraction pattern substantially as shown in FIG. 24;
preferably, the crystal form A of the maleate has a DSC thermogram with an endothermic peak at a temperature of about 119.30 °C;
preferably, the crystal form A of the maleate has a DSC thermogram substantially as shown in FIG. 25;
preferably, the crystal form A of the maleate has a TGA curve substantially as shown in FIG. 26.

9. A crystal form A of the potassium salt of compound I-2 as claimed in claim 1, wherein an X-ray powder diffraction (XRPD) pattern of the crystal form comprises peaks at diffraction angles (2θ) of 11.51±0.2°, 15.42±0.2°, 20.20±0.2°, and 9.52±0.2°;
preferably, the X-ray powder diffraction (XRPD) pattern of the crystal form comprises peaks at diffraction angles (20) of 11.51±0.2°, 15.42±0.2°, 20.20±0.2°, 9.52±0.2°, 5.06±0.2°, and 25.38±0.2°;
preferably, the X-ray powder diffraction pattern of the crystal form A of the potassium salt has the diffraction angles (20) shown in Table 8, wherein the 2θ angles have a margin of error of ±0.20°:
**Table 8**
| 2θ ( ° ) | Intensity% | 2θ ( ° ) | Intensity% |
|---|---|---|---|
| 5.06 | 14.2 | 17.41 | 6.1 |
| 7.67 | 11.2 | 19.14 | 3.1 |
| 9.52 | 15.5 | 20.20 | 25.1 |
| 11.51 | 100.0 | 21.45 | 4.8 |
| 13.86 | 4.0 | 25.38 | 12.0 |
| 15.42 | 59.8 | | |
preferably, the crystal form A of the maleate has the X-ray powder diffraction intensities shown in Table 8;
preferably, the crystal form A of the potassium salt has an X-ray powder diffraction pattern substantially as shown in FIG. 28;
preferably, the crystal form A of the potassium salt has a DSC thermogram with an endothermic peak at a temperature of about 118.44 °C;
preferably, the crystal form A of the potassium salt has a DSC thermogram substantially as shown in FIG. 29;
preferably, the crystal form A of the potassium salt has a TGA curve substantially as shown in FIG. 30.

10. A crystal form A of the magnesium salt of compound I-2 as claimed in claim 1, wherein an X-ray powder diffraction (XRPD) pattern of the crystal form comprises peaks at diffraction angles (2θ) of 13.92±0.2°, 13.46±0.2°, 14.74±0.2°, and 20.43±0.2°;
preferably, the X-ray powder diffraction (XRPD) pattern of the crystal form comprises peaks at diffraction angles (20) of 13.92±0.2°, 13.46±0.2°, 14.74±0.2°, 20.43±0.2°, 20.16±0.2°, and 17.21±0.2°;
preferably, the X-ray powder diffraction pattern of the crystal form A of the magnesium salt has the diffraction angles (20) shown in Table 9, wherein the 2θ angles have a margin of error of ±0.20°:
**Table 9**
| 2θ ( ° ) | Intensity% | 2θ ( ° ) | Intensity% |
|---|---|---|---|
| 4.55 | 9.6 | 15.84 | 17.8 |
| 6.65 | 4.5 | 16.10 | 10.5 |
| 7.14 | 4.7 | 17.21 | 23.0 |
| 7.87 | 4.2 | 18.76 | 8.7 |
| 9.83 | 5.5 | 20.16 | 23.7 |
| 11.04 | 5.7 | 20.43 | 26.3 |
| 11.63 | 6.0 | 20.92 | 11.3 |
| 12.97 | 7.6 | 22.29 | 6.3 |
| 13.46 | 54.8 | 22.63 | 5.9 |
| 13.92 | 100.0 | 24.21 | 4.5 |
| 14.74 | 28.7 | 25.26 | 11.1 |
| 15.37 | 5.9 | 25.89 | 6.6 |
preferably, the crystal form A of the magnesium salt has the X-ray powder diffraction intensities shown in Table 9;
preferably, the crystal form A of the magnesium salt has an X-ray powder diffraction pattern substantially as shown in FIG. 32.

11. A crystal form A of the meglumine salt of compound I-2 as claimed in claim 1, wherein an X-ray powder diffraction (XRPD) pattern of the crystal form comprises peaks at diffraction angles (2θ) of 3.05±0.2°, 9.38±0.2°, 17.62±0.2°, and 12.01±0.2°;
preferably, the X-ray powder diffraction (XRPD) pattern of the crystal form comprises peaks at diffraction angles (20) of 3.05±0.2°, 9.38±0.2°, 17.62±0.2°, 12.01±0.2°, 20.39±0.2°, and 14.88±0.2°;
preferably, the X-ray powder diffraction pattern of the crystal form A of the meglumine salt has the diffraction angles (20) shown in Table 10, wherein the 2θ angles have a margin of error of ±0.20°:
**Table 10**
| 2θ ( ° ) | Intensity% | 2θ ( ° ) | Intensity% |
|---|---|---|---|
| 3.05 | 100.0 | 20.39 | 21.0 |
| 9.38 | 79.6 | 21.71 | 3.7 |
| 12.01 | 21.6 | 22.67 | 4.1 |
| 12.50 | 8.6 | 23.35 | 6.3 |
| 14.88 | 15.0 | 24.41 | 2.6 |
| 16.18 | 12.4 | 26.49 | 2.8 |
| 17.62 | 23.5 | 27.56 | 2.8 |
| 19.53 | 5.0 | 30.15 | 3.0 |
preferably, the crystal form A of the meglumine salt has the X-ray powder diffraction intensities shown in Table 10;
preferably, the crystal form A of the meglumine salt has an X-ray powder diffraction pattern substantially as shown in FIG. 33;
preferably, the crystal form A of the meglumine salt has a DSC thermogram with an endothermic peak at a temperature of about 123.07 °C;
preferably, the crystal form A of the meglumine salt has a DSC thermogram substantially as shown in FIG. 34;
preferably, the crystal form A of the meglumine salt has a TGA curve substantially as shown in FIG. 35.

12. A crystal form A of the tromethamine salt of compound I-2 as claimed in claim 1, wherein an X-ray powder diffraction (XRPD) pattern of the crystal form comprises four or more peaks at diffraction angles (2θ) of 3.68±0.2°, 7.48±0.2°, 17.21±0.2°, and 19.15±0.2°;
preferably, the X-ray powder diffraction (XRPD) pattern of the crystal form comprises peaks at diffraction angles (20) of 3.68±0.2°, 7.48±0.2°, 17.21±0.2°, 19.15±0.2°, 16.73±0.2°, and 15.74±0.2°;
preferably, the X-ray powder diffraction pattern of the crystal form A of the tromethamine salt has the diffraction angles (20) shown in Table 11, wherein the 2θ angles have a margin of error of ±0.20°:
**Table 11**
| 2θ ( ° ) | Intensity% | 2θ ( ° ) | Intensity% |
|---|---|---|---|
| 3.68 | 100.0 | 19.15 | 9.6 |
| 7.48 | 51.3 | 20.18 | 3.3 |
| 14.82 | 7.2 | 21.95 | 3.9 |
| 15.74 | 9.0 | 22.67 | 4.9 |
| 16.73 | 9.2 | 24.66 | 4.0 |
| 17.21 | 12.7 | 27.68 | 4.3 |
preferably, the crystal form A of the tromethamine salt has the X-ray powder diffraction intensities shown in Table 11;
preferably, the crystal form A of the tromethamine salt has an X-ray powder diffraction pattern substantially as shown in FIG. 36;
preferably, the crystal form A of the tromethamine salt has a DSC thermogram with an endothermic peak at a temperature of about 167.96 °C;
preferably, the crystal form A of the tromethamine salt has a DSC thermogram substantially as shown in FIG. 37;
preferably, the crystal form A of the tromethamine salt has a TGA curve substantially as shown in FIG. 38.

13. A preparation method for the pharmaceutically acceptable salt of compound I-1 or compound I-2 and the crystal form A as claimed in any one of claims 1-12, comprising reacting compound I-1 or compound I-2 with an acid or base in a solvent to give the pharmaceutically acceptable salt of compound I-1 or compound I-2, wherein:
preferably, the acid is selected from an inorganic acid and an organic acid, wherein the inorganic acid may be selected from hydrobromic acid, hydrochloric acid, sulfuric acid, sulfurous acid, phosphoric acid, and boric acid; the organic acid may be selected from acetic acid, oxalic acid, valeric acid, benzoic acid, lactic acid, toluic acid, citric acid, malic acid, maleic acid, fumaric acid, succinic acid, tartaric acid, methanesulfonic acid, benzenesulfonic acid, and *p*-toluenesulfonic acid;
preferably, the base is selected from an inorganic base and an organic base, wherein the inorganic base may be selected from alkali metal hydroxides and alkaline earth metal hydroxides, such as sodium hydroxide, lithium hydroxide, potassium hydroxide, calcium hydroxide, and magnesium hydroxide; the organic base may be selected from ammonia, primary amines, secondary amines, and tertiary amines, such as tetramethylamine, tetraethylamine, methylamine, dimethylamine, trimethylamine, triethylamine, ethylamine, meglumine, choline, and tromethamine;
preferably, a molar ratio of compound I-1 or I-2 to the acid or the base may be 1:0.8-1:2; preferably, the molar ratio is 1:0.9-1:1.8.

14. The preparation method as claimed in claim 13, wherein the preparation method for the pharmaceutically acceptable salt of compound I-1 comprises the following methods 1a-1e:
method 1a, comprising: dissolving compound I-1 in acetonitrile, adding concentrated hydrochloric acid, L-tartaric acid, maleic acid, or methanesulfonic acid, stirring at room temperature, then filtering, and drying to give the hydrochloride of compound I-1, the tartrate of compound I-1, the maleate of compound I-1, or the methanesulfonate of compound I-1;
method 1b, comprising: dissolving compound I-1 and citric acid in acetone, stirring at room temperature, then filtering, and drying to give the citrate of compound I-1;
method 1c, comprising: dissolving compound I-1 and sodium hydroxide or potassium hydroxide in acetonitrile or methyl isobutyl ketone, stirring at room temperature, then filtering, and drying to give the sodium salt of compound I-1 or the potassium salt of compound I-1;
method 1d, comprising: dissolving compound I-1 and meglumine in acetonitrile, stirring at room temperature, then filtering, and drying to give the meglumine salt of compound I-1; and
method 1e, comprising: dissolving compound I-1 and tromethamine in N-methylpyrrolidone, adding to toluene, stirring at room temperature, then filtering, and drying to give the tromethamine salt of compound I-1, wherein preferably, a volume ratio of N-methylpyrrolidone to toluene is 2:15;
the preparation method for the pharmaceutically acceptable salt of compound I-2 comprises the following methods 2a-2f:
method 2a, comprising: dissolving compound I-2 and citric acid or L-tartaric acid in acetone, stirring at room temperature, then filtering, and drying to give the citrate of compound I-2 or the tartrate of compound I-2;
method 2b, comprising: dissolving compound I-2 and L-malic acid or fumaric acid in acetonitrile/water, stirring at room temperature, then filtering, and drying to give the malate of compound I-2 or the fumarate of compound I-2, wherein preferably, a volume ratio of acetonitrile to water is 1:1;
method 2c, comprising: dissolving compound I-2 and methanesulfonic acid or maleic acid in ethyl acetate, stirring at room temperature, then filtering, and drying to give the methanesulfonate of compound I-2 or the maleate of compound I-2;
method 2d, comprising: dissolving compound I-2 and sodium hydroxide, potassium hydroxide, calcium hydroxide, or magnesium hydroxide in a mixed solvent of acetonitrile and water or in ethyl acetate, stirring at room temperature, then filtering, and drying to give the sodium salt of compound I-2, the potassium salt of compound I-2, the calcium salt of compound I-2, and the magnesium salt of compound I-2, wherein preferably, a volume ratio of acetonitrile to water is 1:1;
method 2e, comprising: dissolving compound I-2 and meglumine in acetone, stirring at room temperature, then filtering, and drying to give the meglumine salt of compound I-2; and
method 2f, comprising: dissolving compound I-2 and tromethamine in isopropanol, stirring at room temperature, then filtering, and drying to give the tromethamine salt of compound I-2.

15. A pharmaceutical composition, comprising at least one of the pharmaceutically acceptable salt of the compound represented by formula (I) and the crystal form A as claimed in any one of claims 1-12 and a pharmaceutically acceptable carrier.

16. Use of at least one of the pharmaceutically acceptable salt of the compound represented by formula (I) and the crystal form A as claimed in any one of claims 1-12 for the manufacturing of a medicament for the treatment of a metabolic disease, a tumor, an autoimmune disease, or a metastatic disease, wherein:
preferably, the disease is selected from T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, kidney disease, diabetic retinopathy, adipocyte dysfunction, visceral adipocyte accumulation, sleep apnea, obesity, eating disorders, weight gain caused by use of other medicaments, excessive sugar craving, dyslipidemia, hyperinsulinemia, NAFLD, NAS, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome XI, premenstrual syndrome, angina pectoris, thrombosis, atherosclerosis, transient ischemic attacks, vascular restenosis, impaired glucose metabolism, impaired fasting blood glucose conditions, hyperuricemia, gout, erectile dysfunction, skin and connective tissue abnormalities, psoriasis, foot ulcers, ulcerative colitis, hyperapoB lipoproteinemia, Alzheimer's disease, schizophrenia, impaired cognitive function, inflammatory bowel disease, short bowel syndrome, Crohn's disease, colitis, irritable bowel syndrome, polycystic ovary syndrome, and addiction.
